# EUROPÄISCHE PATENTSCHRIFT

(11) **EP 1 761 761 B1**
(45) Veröffentlichungstag und Bekanntmachung des Hinweises auf die Patenterteilung: **16.05.2018**
(21) Anmeldenummer: 05761635.1
(22) Anmeldetag: 29.06.2005
(51) Int. Cl.: G01N 27/403, G01N 27/49, G01N 27/416, G01N 33/543, C12Q 1/68, G01N 27/327

(54) **VORRICHTUNG UND VERFAHREN ZUR EMULATION EINER GEGENELEKTRODE IN EINEM MONOLITHISCH INTEGRIERTEN ELEKTROCHEMISCHEN ANALYSESYSTEM**
PROCESS AND DEVICE FOR EMULATING A COUNTER-ELECTRODE IN A MONOLITHICALLY INTEGRATED ELECTROCHEMICAL ANALYSIS SYSTEM
DISPOSITIF ET PROCEDE D'EMULATION D'UNE CONTRE-ELECTRODE DANS UN SYSTEME D'ANALYSE ELECTROCHIMIQUE MONOLITHIQUEMENT INTEGRE

(30) Priorität: 29.06.2004 DE 102004031370
(43) Veröffentlichungstag der Anmeldung: 14.03.2007
(73) Patentinhaber: Siemens Aktiengesellschaft, 80333 München (DE)
(72) Erfinder: PAULUS, Christian, 82362 Weilheim (DE)
(86) Internationale Anmeldenummer: PCT/DE2005/001146
(87) Internationale Veröffentlichungsnummer: WO 2006/000207

(56) Entgegenhaltungen:
- EP-A- 0 567 725
- US-A1- 2002 039 743
- PAULUS C ET AL: "A fully integrated CMOS sensor system for chronocoulometry" PROCEEDINGS OF IEEE SENSORS 2003. 2ND. IEEE INTERNATIONAL CONFERENCE ON SENSORS. TORONTO, CANADA, OCT. 22 - 24, 2003, IEEE INTERNATIONAL CONFERENCE ON SENSORS, NEW YORK, NY : IEEE, US, Bd. VOL. 2 OF 2. CONF. 2, 22. Oktober 2003 (2003-10-22), Seiten 1329-1332Vol2, XP010690994 ISBN: 0-7803-8133-5
- Michael Kudera ET AL: "Electrochemical Characterisation and Application of Multi Microelectrode Array Devices to Biological Electrochemistry", Sensors, vol. 1, no. 1, 20 June 2001 (2001-06-20), pages 18-28, XP055188678, DOI: 10.3390/s10100018

## Beschreibung

Die Erfindung betrifft eine Vorrichtung und ein Verfahren zur Emulation einer Gegenelektrode in einem monolithisch integrierten elektrochemischen Analysesystem.

Mit einem elektrochemischen Analyseverfahren können Stoffe aufgrund bestimmter physikalischer Eigenschaften unter Ausnutzung des elektrischen Stromes sowohl qualitativ als auch quantitativ bestimmt werden. Von besonderer Bedeutung sind elektrochemische Analyseverfahren bei denen Elektrodenreaktionen eine Rolle spielen. Diese werden, je nachdem ob das Anregungssignal (Strom, Spannung oder Potential) konstant gehalten wird, in zwei Gruppen eingeteilt. Beispielsweise sind die Potentiometrie, Chronopotentiometrie, Coulometrie, Amperometrie, Chronoamperometrie und Chronocoulometrie Techniken, bei denen das Anregungssignal konstant gehalten wird. In voltametrischen und polarographischen Verfahren wird das Anregungssignal variiert.

Gemeinsam mit optischen Verfahren sind derartige elektrochemische Analyseverfahren zur analytischen Bestimmung von chemischen und biochemischen Substanzen durch eine hohe Empfindlichkeit sowie eine hohe Selektivität gekennzeichnet. Während jedoch bei optischen Analyseverfahren aufwendige, teuere und empfindliche optische und optoelektronische Geräte notwendig sind, kommen elektrochemische Analyseverfahren mit vergleichsweise einfachen Elektrodenvorrichtungen aus. Ein entscheidender Vorteil elektrochemischer Analyseverfahren ist das direkte Vorliegen des Messergebnisses als elektrisches Signal. Dieses kann nach einer Analog-Digital-Wandlung direkt von einem Computer, vorzugsweise von einem Personalcomputer, weiterverarbeitet werden.

Elektrochemische Analyseverfahren eignen sich zur qualitativen wie quantitativen Messung von Stoffkonzentrationen in einer Elektrolytlösung. Jeder Stoff weist eine für ihn charakteristische Oxydationsspannung bzw. Reduktionsspannung auf. Anhand dieser Spannungen kann zwischen verschiedenen Stoffen unterschieden werden. Ferner kann aufgrund des während einer Reaktion geflossenen elektrischen Stromes auf die Konzentration eines vorliegenden Stoffes geschlossen werden.

Ein elektrochemisches Experiment erfordert mindestens zwei Elektroden, die mit dem zu analysierenden Stoff (Eletrolyt) in Verbindung stehen (Arbeitselektrode, Gegenelektrode). Es können jedoch auch mehrere Arbeitselektroden parallel verwendet werden. Zur exakten Kontrolle des Elektrolytpotentials wird zumeist noch eine Referenzelektrode eingesetzt. Dieses mindestens drei Elektroden aufweisende System wird mit einem Potentiostaten verbunden, der die Regelung des Potentials an der Arbeitselektrode ermöglicht und den durch die Arbeitselektrode fließenden elektrischen Strom misst.

Bei der Voltametrie wird an die Arbeitselektrode eine veränderliche Spannung angelegt und der bei einer Oxydation oder Reduktion fließende Strom gemessen. Im Spezialfall der Cyclovoltametrie wird ein bestimmter Spannungsbereich wiederholt derart überstrichen, dass die Inhaltsstoffe des Elektrolyten mehrmals hintereinander oxydiert und reduziert werden.

Bei der Chronoamperometrie wird an die Arbeitselektrode sprunghaft eine definierte Spannung angelegt und der fließende Strom über die Zeit aufgezeichnet. Diese Meßmethode erlaubt die Analyse eines bestimmten Stoffes durch die gezielte Oxydation oder Reduktion dieses Stoffes. Der geflossene Strom ist ein Maß für die je Zeiteinheit umgesetzte Stoffmenge und lässt Rückschlüsse auf die Konzentration des Stoffs und der Diffusionskonstanten zu.

Die Chronocoulometrie entspricht in den elektrischen Randbedingungen der Chronoamperometrie. Im Unterschied dazu wird jedoch nicht der geflossene elektrische Strom, sondern die insgesamt geflossene elektrische Ladung aufgezeichnet.

In der Ausgestaltung als Sensoren können Elektrodenvorrichtungen in verschiedenen elektrochemischen Analyseverfahren eingesetzt werden. Entscheidend ist lediglich, dass beim Senorereignis elektrochemisch auswertbare Stoffe erzeugt werden. Bei Sensoren zur Detektion von Biomolekülen bedient man sich beispielsweise eines Markierungsverfahrens, das im Falle eines Sensorereignisses elektrochemisch aktive Stoffe bereitstellt.

Miniaturisierte elektrochemische Elektrodensysteme zur Analyse chemischer und biochemischer Substanzen sind im Stand der Technik bekannt [1], [2], [3], [4] und [5]. Die Elektroden derartiger Arrays können am Rande des Substrats einzeln kontaktiert und mittels eines Potentiostaten betrieben werden. Um zudem Elektrodenarrays zu realisieren, die beispielsweise 100 oder mehr Elektroden aufweisen, sind Schaltfunktionen auf dem Substrat vorteilhaft, die die Elektroden auf gemeinsame Anschlussleitungen multiplexen. Ist das Substrat ein Halbleitermaterial wie Silizium, können die notwendigen Schalter durch MOS-Transistoren realisiert werden, wie in [6] beschrieben. Aufgrund der dadurch erreichbaren Parallelisierung der Tests wird die Analysezeit wesentlich verkürzt und auch die Durchführung komplexer Analysen möglich.

In [7] ist das so genannte EDDA-Verfahren (Electrically Detected Displacement Assay-Verfahren) der Firma Friz BIOCHEM™ beschrieben.

Aus Sicht der Miniaturisierung, der Signalintegrität sowie der Messempfindlichkeit stellen die im Stand der Technik bekannten aktiven Microarrays sehr gute elektrochemische Analysesysteme dar [2]. Hier sind in dem Halbleitermaterial nicht nur die Multiplex- bzw. Auswahlfunktionen, sondern auch die Verstärkung, die Konditionierung der Signale und gegebenenfalls auch die Auswertung der Signale integriert. Diese Sensorarrays werden als aktive Arrays bezeichnet, da im Gegensatz zu den passiven Arrays aktive Elektronik Signale auf dem Chip verarbeitet. Derartige aktive elektrochemische Sensorarrays, die gemäß voltametrischer, (chrono-)amperometrischer und (chrono-)coulometrischer Verfahren arbeiten, werden gemäß der CMOS-Technologie gefertigt und mit auf der Chipoberfläche zugänglichen Elektroden aus einem Edelmetall (z.B. Gold) ausgestattet.

Aktive Sensorarrays sind beispielsweise bei DNA-Sensorchips verwirklicht worden, bei denen auf Basis des Redox-Cycling DNA-Moleküle an Oberflächen elektronisch durch Erfassen von mittels redoxaktiven Stoffen erzeugten elektrischen Ladungsträgern nachgewiesen werden. Das Redox-Cycling stellt einen Spezialfall eines amperometrischen Verfahrens dar (Oxidations-/Reduktionsspannungen konstant, Messungen des Elektrodenstroms) .

Eine typische Redox-Cycling-Sensor-Anordnung weist zwei auf einem Substrat ausgebildete Gold-Elektroden auf. Eine solche Sensoranordnung offenbart beispielsweise Kudera M. et al. in "Electrochemical Characterization and Application of Multi Microelectrode Array Devices to Biological Electrochemistry", Sensors, Bd. 1, Nr. 1, 20. Juni 2001, Seiten 18 bis 28. Auf jeder Elektrode sind beispielsweise über die sogenannte Gold-Schwefel-Kopplung immobilisierte einzelsträngige DNA-Fängermoleküle mit einer vorgegebenen Sequenz immobilisiert. Die gegebenenfalls in der Analyselösung vorliegenden komplementären und damit zur Hybridisierung befähigten einzelsträngigen DNA-Zielmoleküle weisen eine Markierung auf. Mittels dieser Markierung wird bei Anwesenheit geeigneter Zusatzmoleküle ein Zyklus aus Oxydation und Reduktion von Bestandteilen der Zusatzmoleküle ausgelöst, der unter Wechselwirkung mit den Elektroden zur Bildung reduzierter bzw. oxydierter Moleküle führt. Der Zyklus aus Oxidationen und Reduktionen führt zu einem elektrischen Kreisstrom, der einen Nachweis der DNA-Zielmoleküle ermöglicht.

Weiterhin offenbaren die EP 0 567 725 A1 ein Verfahren zum Verbessern der Lebensdauer eines implantierbaren Sensors und die US 2002/0039743 A1 einen Nukleinsäuredetektionssensor. Schließlich offenbart Paulus et al. in "A Fully Integrated CMOS Sensor System for Chronocoulometry", Proceedings of IEEE Sensors 2003, 2nd IEEE International Conference on Sensors, Toronto, Canada, 22. bis 24. Oktober 2003, Bd. Vol. 2 of 2, Conf. 2, Seiten 1329 bis 1332, Vol. 12.

Sowohl bei dieser Redox-Cycling-Sensor-Anordnung als auch bei den zuvor genannten elektrochemischen Analyseverfahren ist stets eine Gegenelektrode erforderlich. Während bei den Redox-Cycling-Sensoren jedoch nur ein relativ kleiner Gleichstrom an den Elektroden abgeführt werden muss, muss bei den meisten der oben angegebenen elektrochemischen Analyseverfahren ein vergleichsweise hoher Stoßstrom durch die Gegenelektrode geliefert werden können. Aus diesem Grund muss die Fläche der Gegenelektrode wesentlich größer sein als die der aktiven Arbeitselektroden. In Abhängigkeit vom konkreten Analyseverfahren ist für die Gegenelektrode regelmäßig eine etwa 10-fach größere Oberfläche in Bezug auf die Summe der Oberflächen der einzelnen Arbeitselektroden zu fordern. Das ist notwendig, weil bei einer zu kleinen Fläche der Gegenelektrode die an ihr anliegende Spannung bei einem Experiment zur Bereitstellung der erforderlichen Ladungsträger extrem hohe Werte annehmen kann. Sofern derartig hohe Werte angenommen werden, können unkontrolliert ablaufende chemische Umsetzungen, z. B. des Elektrodenmaterials die Folge sein, die typischerweise unter Bildung von Gasen erfolgen.

Ist die Oberfläche der Gegenelektrode groß genug, ist sie in der Lage, das Elektrolytpotential größtenteils durch die Doppelschichtkapazität zu stabilisieren. Elektrochemische Umsetzungen finden nur mit vergleichsweise niedrigen Stromdichten statt.

Da ein aktiver Siliziumchip als Substrat z.B. für einen DNA-Sensor vergleichsweise teuer ist, wird im allgemeinen eine möglichst hohe Packungsdichte der Einzelsensoren im Array angestrebt. Wegen der Packungsdichte der Sensoren und damit der Elektroden kann im Bereich des Sensorarrays unter Umständen keine Gegenelektrode realisiert werden. Die Gegenelektrode kann dann als externe Elektrode ausgeführt sein, die im Probenvolumen angeordnet und mit dem Sensorchip elektrisch verbunden ist. Die Ansteuerung dieser Elektrode kann von einem Potentiostaten übernommen werden. Wegen vergleichsweise langer Zuleitungen und dem aufwendigeren mechanischen Aufbau ist diese Vorgehensweise allerdings nachteilhaft. Sofern die damit verbundenen Nachteile vermieden werden sollen, bietet der Stand der Technik als Lösung lediglich an, dass die Gegenelektrode in der Peripherie des Arrays realisiert wird, was jedoch zusätzliche (teuere) Chipfläche erfordert.

Der Erfindung liegt insbesondere das Problem zugrunde, ein Sensor-Array sowie ein Verfahren zum Betreiben eines Sensor-Arrays bereitzustellen, wobei die aus dem Stand der Technik bekannten Probleme bei der Realisierung einer Gegenelektrode vermindert werden.

Das Problem wird durch ein Sensor-Array sowie durch ein Verfahren zum Betreiben eines Sensor-Arrays mit den Merkmalen gemäß den unabhängigen Patentansprüchen gelöst. Bevorzugte Weiterbildungen der Erfindung ergeben sich aus den abhängigen Ansprüchen.

Das erfindungsgemäße Sensor-Array weist mindestens drei Elektroden und eine Schalteinheit auf, wobei die mindestens drei Elektroden mittels der Schalteinheit wahlweise als Gegenelektrode oder als mit einem elektrolytischen Analyten elektrisch koppelbare Arbeitselektrode geschaltet werden kann und wobei die mindestens drei Elektroden derart eingerichtet sind, dass an einer als Arbeitselektrode geschalteten Elektrode der mindestens drei Elektroden in der Elektrolytlösung bei Anwesenheit des elektrolytischen Analyten Sensorereignisse stattfinden.

Die Deaktivierung der Betriebsschaltung für die als Sensorelektrode fungierende Elektrode und das Herstellen einer Verbindung zwischen der Elektrode und dem Gegenelektrodenanschluss eines Potentiostaten wird durch die Realisierung einer Schalteinheit realisiert.

Die Elektrode einer Sensor-Anordnung ist derart eingerichtet, dass an der als Arbeitselektrode geschalteten Elektrode bei Anwesenheit des nachzuweisenden elektrolytischen Analyten Sensorereignisse stattfinden. Die Gegenelektrode des Gesamtsystems, d.h. des Sensor-Arrays, wird durch Zusammenschalten der mit dem Gegenelektrodenanschluß des Potentiostaten verbundenen Elektroden, deren Betriebsschaltungen als Sensorelektroden deaktiviert wurden, realisiert.

Auf diese Weise kann die für elektrochemische Experimente notwendige Gegenelektrode durch Zusammenschalten unbenutzter Sensorelektroden eines Sensor-Arrays realisiert werden. Eine separate und flächenintensive Gegenelektrode wird somit entbehrlich und insbesondere monolithisch integrierte elektrochemische Analysensysteme können, da keine teure Chipfläche für die Realisierung einer Gegenelektrode bereitgestellt werden muss, kostengünstiger hergestellt werden.

Unter Arbeitselektrode wird insbesondere eine Elektrode verstanden, die mit einem elektrolytischen Analyten gekoppelt ist und an der die für ein Sensorereignis relevanten, beispielsweise elektrochemischen Umsetzungen erfolgen.

Unter einer Gegenelektrode wird insbesondere eine Elektrode verstanden, die mit einem elektrolytischen Analyten gekoppelt ist und diesem bedarfsweise elektrische Ladungsträger bereitstellt, um ein vorgegebenes elektrochemisches Potential des Analyten einzustellen.

Das erfindungsgemäße Sensor-Array ist derart realisiert, dass dieses einen Steuerschaltkreis aufweist, der zum Ansteuern, Auswählen und/oder Auslesen der Sensor-Elektroden oder eines Teils der Sensor-Elektroden (beispielsweise der Elektroden einer Zeile oder Spalte von Sensor-Pixeln) eingerichtet ist. Ein solcher Steuerschaltkreis, der auf und/oder in einem Chip integriert sein kann oder von dem Chip extern vorgesehen sein kann, enthält beispielsweise eine Vielzahl von Auswahltransistoren, Zeilenleitungen und Spaltenleitungen, um gezielt einzelne Sensor-Pixel anzusteuern, auszuwählen bzw. ein Sensorsignal auszulesen.

Vorzugsweise ist der mit der Elektrode bei Schaltung als Arbeitselektrode gekoppelte Betriebsschaltkreis derart eingerichtet, dass er im Fall von Sensorereignissen ein die Sensorereignisse charakterisierendes elektrisches Sensorsignal bereitstellt. Dieses Sensorsignal kann beispielsweise ein Sensorstrom oder eine Sensorspannung sein. Das Sensorsignal kann auch on-chip vorverarbeitet, z.B. digitalisiert und/oder verstärkt werden, um das Signal/Rausch-Verhältnis zu verbessern. Das erfindungsgemäße Sensor-Array ist derart ausgestaltet ist, dass die einzelnen Sensorelektroden monolithisch in und/oder auf einem Substrat integriert sind. Das Substrat kann beispielsweise ein Halbleitersubstrat, insbesondere ein Siliziumsubstrat (wie ein Siliziumwafer oder ein Siliziumchip) sein. Dadurch können zur Ansteuerung der Sensorelektroden miniaturisierte integrierte Schaltkreise ausgebildet werden. Der oder die Teilschaltkreise für eine Elektrode, beispielsweise der Betriebsschaltkreis, kann unterhalb des elektrochemischen Systems mit den Elektroden vorgesehen sein, was eine besonders platzsparende Konfiguration ermöglicht. Alternativ kann zumindest ein erster Teil der Komponenten des Schaltkreises extern von (d.h. getrennt von) einem Substrat vorgesehen sein, in und/oder auf dem ein zweiter Teil der Komponenten der Sensor-Anordnung ausgebildet ist.

Die Schaltkreise zur Ansteuerung der Elektroden im erfindungsgemäßen Sensor-Array müssen derart eingerichtet sein, dass die Betriebsschaltung für die Sensorelektrode deaktivierbar ist. Deaktivierbar bedeutet in diesem Zusammenhang, dass im Inaktiv-Zustand keine Ladungsträger aus der Betriebsschaltung in die Arbeitselektrode fließen können. Dies kann entweder durch ein völliges Abkoppeln der Elektrode mittels eines Schalters (Schalttransistor, Transmission Gate) erfolgen oder durch die Wahl eines geeigneten Betriebspunktes der Arbeitselektrodenschaltung, der die obige Forderung erfüllt. Zudem muss die Sensorelektrode innerhalb des Sensor-Arrays mit einer Schalteinheit verbunden sein, die die Verbindung zwischen der Elektrode und dem Gegenelektrodenanschluss des Potentiostaten herstellt. Diese Schalteinheit weist bevorzugt mindestens einen MOS-Transistor auf.

Alternativ kann die Betriebsschaltung der als Gegenelektrode geschalteten Sensorelektroden auch derart angesteuert werden, dass das Elektrodenpotential dem Potential am Gegenelektrodenanschluss des Potentiostaten folgt. In diesem Falle ist nicht zwingend die Realisierung eines Schalters im Sensor-Pixel erforderlich. Die Schalteinheit kann auch einen Schalter aufweisen, der am Rand der Matrix liegt und mehrere Sensor-Pixel ansteuert, beispielsweise alle Sensor-Pixel einer Spalte.

Die Elektroden in dem erfindungsgemäßen Sensor-Array können zur Erfassung von oxidierbaren bzw. reduzierbaren Stoffen eingerichtet sein. Diese Sensor-Arrays können 50 bis 1000000, bevorzugt 100 bis 250000 einzelne Sensor-Elektroden aufweisen. Bei dem erfindungsgemäßen Sensor-Array können die Sensor-Pixel im Wesentlichen matrixförmig, das heisst in Zeilen und Spalten, angeordnet sein. Dies ermöglicht eine besonders hohe Integrationsdichte der Sensor-Pixel, was insbesondere für High-Throughput-Analysen vorteilhaft ist, bei denen jede Sensor-Elektrode auf ein anderes Biomolekül sensitiv ist, beispielsweise auf Oligonukleotide mit unterschiedlicher Basensequenz.

Eine bevorzugte Ausführungsform der Erfindung betrifft einen Sensor-Array in dem die Elektroden zum Erfassen von Biomolekülen eingerichtet sind. Diese Biomoleküle sind beispielsweise Nukleinsäuremoleküle, Peptide und/oder Proteine. Die Elektroden innerhalb des Sensor-Arrays sind bevorzugt derart eingerichtet, dass auf der Oberfläche der Elektroden Fängermoleküle immobilisiert sind. Diese Fängermoleküle sind in der Lage, die zu detektierenden Biomoleküle zu erkennen und diese**n** spezifisch zu binden.

Beispielsweise können Elektroden zur Analyse von DNA-Molekülen durch die Immobilisierung von einzelsträngigen DNA-Fragmenten, die gegenüber den zu detektierenden DNA-Molekülen komplementär und damit zur spezifischen Hybridisierung befähigt sind, funktionalisiert sein. Eine derartige funktionalisierte Schicht kann gegebenenfalls auch die elektrochemischen Markierungen für die anschließende Detektion bereitstellen.

Es ist bevorzugt, dass die immobilisierten Fängermoleküle Ferrocen-Markierungen aufweisen. Das dieser Markierung zugrundeliegende Detektionsprinzip basiert auf der Oxydation von Ferrocen-Markierungen (oxydativer Spannungssprung), wobei die Arbeitselektrode auf ein geeignetes positives Potential gegenüber dem Potential des Elektrolyten gebracht wird. Die Menge der beim Spannungssprung geflossenen elektrischen Ladung ist ein direktes Maß für die Menge an Ferrocen, das an der betreffenden Elektrode vorhanden ist.

Bei dem Sensor-Array der Erfindung kann in Anwesenheit eines elektrolytischen Analyten mittels elektrochemisch aktiver Markierungen ein Sensorsignal generiert werden. Die elektrochemisch aktiven Markierungen können Metallkomplex-Markierungen aufweisen. Die elektrochemisch aktiven Markierungen können Ferrocen-Markierungen aufweisen. Solche elektrochemisch aktiven Markierungen können an Fängermolekülen, Zielmolekülen, Intercalatoren oder Signal-Oligomeren angebracht sein.

Wird eine derartige Elektrode nun erfindungsgemäß auch als Gegenelektrode benutzt, so muss sichergestellt sein, dass die Funktionalisierung hierdurch nicht zerstört wird. Dies ist gewährleistet, wenn die Gesamtfläche der als Gegenelektrode geschalteten Sensorelektroden wesentlich größer ist als die Fläche der aktiven Sensorelektroden. In diesem Fall nimmt die Spannung an der Gegenelektrode keine extremen Werte an. Es ist daher bevorzugt, dass die als Gegenelektrode zusammengeschalteten Elektroden des Sensor-Arrays insgesamt etwa die 10- bis 100-fache Fläche der als Arbeitselektrode(n) geschaltete(n) Elektrode(n) aufweisen. Dies ist bevorzugt, da dann eine Potentialverschiebung des Elektrolyten im Wesentlichen auf der kapazitiven Kopplung der Gegenelektrode zum Elektrolyten über Doppelschichtkapazität erfolgt und nur vernachlässigbar wenig Umsetzungen an der Gegenelektrode auftreten. Der Spannungshub, den ein Potentiostat an der Gegenelektrode hervorruft, hat in diesem Fall eine vernachlässigbar kleine Amplitude.

Im Falle der Oxydation von Ferrocen-Markierungen wird die Gegenelektrode beim Spannungssprung gegenüber dem Elektrolyten leicht negativ polarisiert. Die dort immobilisierten_Ferrocen-Moleküle können dadurch also nicht oxydiert werden. Bereits oxydierte Markierungen werden unter diesen Umständen wieder reduziert. In jedem Fall bleibt die Funktionalisierung der als Gegenelektrode verwendeten Sensorelektroden intakt. Das Gleiche gilt entsprechend für den Fall, dass auf der Elektrodenoberfläche Markierungen vorhanden sind, die mittels eines reduktiven Spannungssprungs detektiert werden.

Ferner können die Sensor-Elektroden des erfindungsgemäßen Sensor-Arrays als dynamische Biosensor-Anordnungen eingerichtet sein. Unter einer dynamischen Biosensor-Anordnung wird insbesondere eine solche Biosensor-Anordnung verstanden, die nicht nur quasi-statisch betrieben wird, sondern bei der dynamische, das heisst zeitlich stark veränderliche Meßsignale auftreten (z.B. Spannungssprünge, Wechselspannungs-Voltametrie, etc.)

Gegenstand der Erfindung ist zudem ein Verfahren zum Betreiben des oben beschriebenen Sensor-Arrays in einem elektrochemischen Analyseverfahren.

Nach dem erfindungsgemäßen Verfahren wird in einem ersten Schritt zunächst eine erste Anzahl der mindestens drei Elektroden, die aus mindestens zwei Elektroden besteht, zusammengeschaltet, mit dem Gegenelektrodenanschluß eines Potentiostaten verbunden und hierdurch die Gegenelektrode des Gesamtsystems realisiert. Gleichzeitig ist eine zweite Anzahl der mindestens drei Elektroden, die aus mindestens einer Elektrode besteht, als Arbeitselektrode(n) zur Detektion eines elektrolytischen Analyten geschaltet.

Nach der Erfassung der Sensorereignisse an den genannten Elektroden, die als Arbeitselektroden geschaltet sind, wird in einem weiteren Schritt mindestens eine der zuvor als Gegenelektrode geschalteten Elektrode**n** vom Gegenelektrodenanschluss des Potentiostaten abgetrennt und als Arbeitselektrode geschaltet. Gleichzeitig wird die Betriebsschaltung mindestens einer im ersten Schritt als Arbeitselektrode geschalteten Elektrode deaktiviert und als Teil der Gegenelektrode des Sensor-Arrays mit dem Gegenelektrodenanschluss des Potentiostaten verbunden.

Die Abfolge der zuvor genannten Verfahrensschritte, d.h. der Wechsel zwischen der Schaltung einer Elektrode des Sensor-Arrays als Arbeitselektrode und als Gegenelektrode und umgekehrt kann beliebig oft wiederholt werden.

Im Ergebnis macht sich das erfindungsgemäße Verfahren die Möglichkeit der flexiblen aktiven Ansteuerung der einzelnen Arbeitselektroden im Sensor-Array zunutze. Da nicht alle im Sensor-Array vorhandenen Sensor-(Arbeits-)elektroden gleichzeitig als Elektroden zur Detektion des Analyten verwendet werden, kann der Rest der Elektroden gemeinsam als Gegenelektrode verschaltet werden.

Mittels dieser Vorgehensweise kann dem Erfordernis einer großflächigen Gegenelektrode leicht entsprochen werden. Regelmäßig werden die Sensorpixel ohnehin nur sukzessive ausgelesen, so dass ein gleichzeitiger Betrieb als Arbeitselektrode nicht notwendig ist. So können beispielsweise die Sensoren einer Zeile oder einer Spalte des Sensor-Arrays als aktive Sensoren verwendet werden. Alle anderen Sensorelektroden können zusammengeschaltet als Gegenelektrode des Gesamtsystems arbeiten. Dem Fachmann ist zudem klar, dass auch zufällig bzw. unregelmäßig ausgewählte Sensoren des Sensor-Arrays in Bezug auf ihre Betriebsschaltung deaktiviert werden können und dann nur diese Elektroden als Gegenelektrode fungieren können.

Das erfindungsgemäße Verfahren ist unabhängig von der Form der jeweiligen Elektroden. Vorzugsweise sind diese flächig ausgeführt. Es sind jedoch auch segmentierte Elektroden geeignet. Vorzugsweise erfolgt das Zusammenschalten der nicht als Sensorelektroden aktivierten Elektroden in einem Umfang, dass die Gesamtoberfläche der als Gegenelektroden geschalteten Elektroden mindestens 10fach so groß ist, wie die Gesamtoberfläche der als Arbeitselektroden geschalteten Elektroden.

Es ist zudem bevorzugt, dass in dem erfindungsgemäßen Verfahren zum Betreiben eines Sensor-Arrays elektrochemische Analyseverfahren eingesetzt werden, bei denen mindestens ein Stoff in einem Elektrolyten qualitativ und/oder quantitativ aufgrund dessen charakteristischer Oxydationsspannung bzw. Reduktionsspannung mittels Voltametrie, Amperometrie und/oder Coulometrie bestimmt wird.

Vorzugsweise kann das erfindungsgemäße Verfahren zur Analyse von Biomolekülen verwendet werden. Hierzu können die Elektroden beispielsweise mit immobilisierten Ferrocenmarkierten Fängermoleküle**n** versehen sein und die Konzentration der Biomoleküle durch Messen der bei der Oxydation des Ferrocens fließenden Ladungsmenge bestimmt werden.

Grundsätzlich ist es auch möglich, neben der Nutzung des erfindungsgemäßen Verfahrens, den Chip zusätzlich mit einer konventionellen Gegenelektrode auszurüsten. Dies kann das Zeitverhalten des Gesamtsystems deutlich verbessern.

Im erfindungsgemäßen Verfahren wird die Betriebsschaltung einer Arbeitselektrode vor dem Umschalten in den Betrieb als Gegenelektrode mittels einer Schalteinheit deaktiviert. Für die Detektion des Analyten werden lediglich einige Elektroden des Sensor-Arrays als Arbeitselektroden geschaltet. Als aktive Sensoren, die die oben beschriebene Messung durchführen, werden beispielsweise nur die Sensoren einer Spalte des Sensor-Arrays ausgewählt. Alle anderen Elektroden (z.B. alle anderen Spalten) werden hingegen mittels geeigneter integrierter Schalttransistoren miteinander verbunden und dem Potentiostaten als Gegenelektrode zur Verfügung gestellt. Diese als Gegenelektrode fungierenden Elektroden arbeiten nicht im Sensorbetrieb und man erhält keine Information über die Sensorereignisse aus diesen Sensor-Pixeln.

Ein Aspekt der Erfindung kann anschaulich darin gesehen werden, dass ein monolithisch integriertes elektrochemisches Analysesystem für eine sehr empfindliche und hochgradig parallele Detektion elektrochemisch aktiver Spezies mittels Voltammetrie, Chronoamperometrie und/oder Chronocoulometrie bereitgestellt wird.

Insbesondere das oben beschriebene Verfahren des Potentiostatenbetriebs, bei dem das Arbeitselektrodenpotential konstant gehalten wird und stattdessen das elektrische Potential des Elektrolyten verändert wird, weist in den oben beschriebenen Fällen erhebliche Vorteile auf.

Ferner ist die erfindungsgemäße Sensor-Anordnung für sogenannte Spannungssprungexperimente aufgrund der geeigneten schaltungstechnischen Maßnahmen mit den in die Sensor-Anordnung monolithisch integrierten elektrischen Schaltungen sehr vorteilhaft, um die Stabilität der Sensor-Anordnung während des Spannungssprungexperiments zu gewährleisten.

Die monolithische Integration von Elektroden und Betriebsschaltung ermöglicht die Realisierung hochdichter, hochempfindlicher Sensor-Arrays unter Einhaltung der für die Spannungssprungexperimente erforderlichen hohen Flankensteilheit.

Ausführungsbeispiele der Erfindung sind in den Abbildungen dargestellt und werden im Weiteren näher erläutert.

Es zeigen:
- Figur 1: eine schematische Darstellung einer Anordnung einer Vielzahl von Sensor-Pixeln gemäß einem Aspekt der Erfindung;
- Figur 2: Pixelschaltung gemäß einem Aspekt der Erfindung;
- Figur 3: Pixelschaltung gemäß einem anderen Aspekt der Erfindung;
- Figur 4: eine Pixelschaltung gemäß einem Aspekt der Erfindung;
- Figur 5: eine Pixelschaltung gemäß einem anderen Aspekt der Erfindung;
- Figur 6: die Pixelschaltung gemäß Figur 4 in vereinfachter Darstellung;
- Figuren 7a und 7b: Pixelschaltungen mit Referenzschaltungen gemäß unterschiedlichen Aspekten der Erfindung;
- Figur 8: eine Pixelschaltung gemäß einem anderen Aspekt der Erfindung;
- Figur 9: eine schaltungstechnische Realisierung einer Betriebsschaltung mit einer Vorlade-Schaltung zum Vorladen der Stromspiegel;
- Figur 10: eine schaltungstechnische Realisierung der Potentiostatenschaltung gemäß einem Aspekt der Erfindung; und
- Figur 11: eine schaltungstechnische Realisierung der Pixelschaltung gemäß Figur 6.

Gleiche oder ähnliche Komponenten in unterschiedlichen Abbildungen sind mit gleichen Bezugsziffern versehen.

Die Darstellungen in den Figuren sind schematisch und nicht maßstäblich.

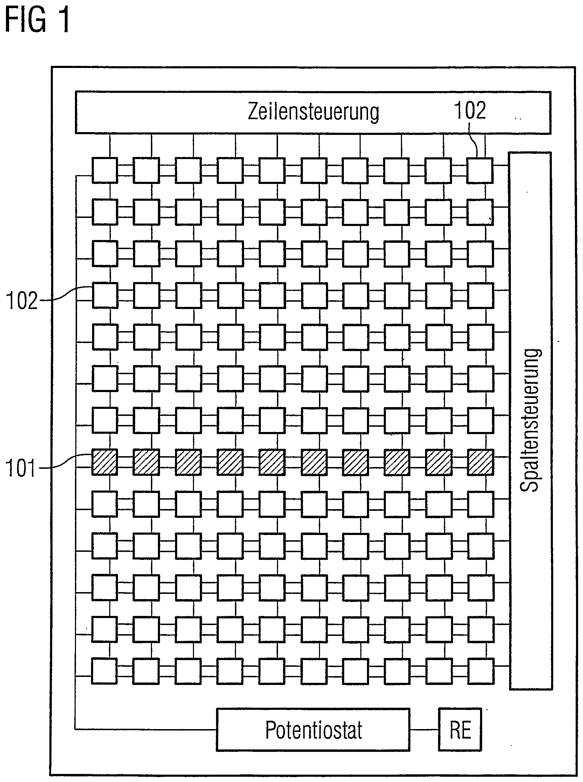

Die in **Fig. 1** dargestellte Pixelanordnung zeigt beispielhaft die Auswahl der Sensoren einer Spalte 101 als aktive Sensoren, während die Elektroden aller anderen Sensor-Anordnungen 102 (alle anderen Spalten) z.B. mittels geeigneter integrierter Schaltransistoren verbunden sind und dem Potentiostaten als Gegenelektrode zur Verfügung gestellt werden. Die Potentiostatenschaltung kann derart eingerichtet und mit der Gegenelektrode gekoppelt sein, dass das vorgegebene Potential als konstantes Elektrolytpotential bereitgestellt wird. Die Potentiostatenschaltung vergleicht die Elektrolytspannung, die ihr von der Referenzelektrode (RE) bereitgestellt wird, mit einer fest vorgegebenen Referenzspannung und steuert die Gegenelektrode derart an, dass das Elektrolytpotential im Wesentlichen konstant bleibt.

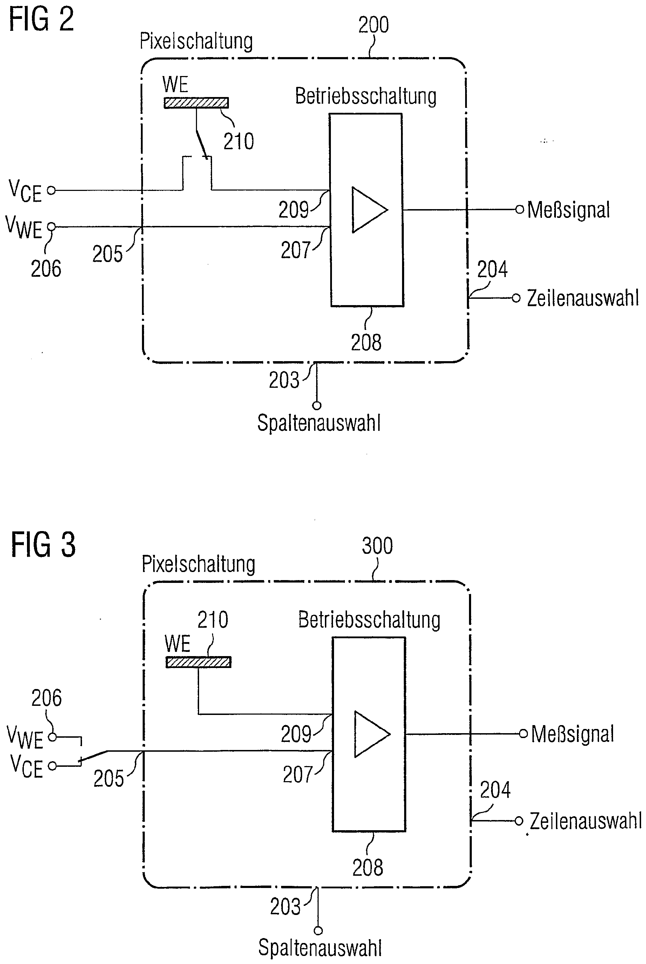

**Fig. 2** zeigt eine Pixelschaltung 200 gemäß einer Ausführungsform der Erfindung, wobei das die Verbindung zwischen der Elektrode und dem Gegenelektrodenanschluss des Potentiostaten herstellende Schaltelement innerhalb des Sensor-Pixels realisiert ist. Jede Pixelschaltung ist mit der jeweiligen Arbeitselektrode zum Erfassen des an der Arbeitselektrode auftretenden Sensorereignisses elektrisch gekoppelt. Der Spaltenauswahl-Eingang 203 ist mit der Spaltensteuerung aus Fig. 1 gekoppelt. Über den Spalten-Auswahleingang 203 wird der Pixelschaltung 200 das Spaltenauswahlsignal zugeführt. Ein zweiter Eingang 204, auch als Zeilen-Auswahl-Eingang 204 bezeichnet, ist mit der in Fig. 1 gezeigten Zeilensteuerung gekoppelt und dient zur Zuführung des Zeilenauswahl-Signals zu der Pixelschaltung 200. Ferner ist ein dritter Eingang 205 zum Zuführen eines Referenzpotenzials V_{WE} 206 an einem ersten Eingang 207 eines ebenfalls in der Pixelschaltung 200 vorgesehenen Operationsverstärkers 208 vorhanden. Der zweite Eingang 209 des Operationsverstärkers 208 ist mit der Arbeitselektrode 210 der jeweiligen Pixelschaltung 200 gekoppelt.

**Fig. 3** gibt eine alternative Pixelschaltung 300 gemäß einer weiteren Ausführungsform der Erfindung wieder. Diese Art der Schaltung einer Elektrode im Sensor-Array sieht von einem Schalter im Sensor-Pixel ab und verwirklicht anstelle dessen eine mindestens einen Schalter umfassende Schalteinheit am Rand der Matrix, die mehrere Pixel, z.B. alle Pixel einer Spalte, ansteuert.

Die im Folgenden beschriebenen Pixelschaltungen sind jeweils unter einer Sensorelektrode, wie sie beispielsweise in Fig.1 dargestellt ist, angeordnet und monolithisch in das Siliziummaterial integriert, so dass die im Folgendem beschriebenen jeweiligen Schaltungskomponenten gebildet werden und miteinander verschaltet sind, so dass die im Folgenden beschriebenen Funktionalitäten gewährleistet sind.

Die im Folgenden beschriebenen Sensor-Anordnungen sind als DNA-Sensoren eingerichtet zum Erfassen von makromolekularen Biopolymeren, wobei zum Erfassen der makromolekularen Biopolymere gemäß diesen Ausführungsbeispielen der Erfindung ein elektrochemisches Detektionsverfahren eingesetzt wird, bei dem ein indirekter Nachweis eines Hybridisierungsereignisses mittels signalgebender elektrochemischer Markierungen am Fängermolekül, am Zielmolekül, an Signal-Oligomeren oder durch Intercalatoren erfolgt. Intercalatoren sind insbesondere Stoffe, die sich selektiv an bzw. in doppelsträngiger DNA einlagern. Dies erlaubt eine Diskriminierung zwischen Einzelsträngen bzw. Doppelsträngen.

Gemäß dem in [7] beschriebenen Verfahren sind kurzkettige Signal-Oligomere mit den auf den Arbeitselektroden immobilisierten Fängermolekülen (Probe-Moleküle) hybridisiert. Anschließend wird die zu analysierende Substanz mit dem elektronischen Chip in Kontakt gebracht und in der zu analysierenden Substanz eventuell vorhandene, zu den jeweiligen Probe-Molekülen, auch bezeichnet als Fängermoleküle, in ihrer Sequenz komplementäre Ziel-Moleküle verdrängen die kurzkettigen Signal-Oligomere. An den Signal-Oligomeren sind üblicherweise Ferrocen-Moleküle als Markierung angebracht. Das elektrochemische System detektiert anschließend das Vorhandensein der Ferrocen-Moleküle an den verschiedenen Sensorpositionen.

In diesem Zusammenhang ist anzumerken, dass die im Folgenden beschriebenen Sensor-Anordnungen für alle Arten von elektrochemischen Experimenten geeignet sind, bei denen eine Oxidation und/oder eine Reduktion von Inhaltsstoffen des Elektrolyten oder von auf der Elektrodenoberfläche immobilisierten Stoffen durchgeführt wird.

Die folgenden Ausführungsformen beschreiben somit ein DNA-Analysesystem für die Chronoamperometrie und Chronocoulometrie, das allgemein in der Lage ist, einen Spannungssprung von mehreren 100 mV (typischerweise 400 mV) innerhalb einer Zeitspanne von weniger als einer Mikrosekunde, prinzipiell aber auch beliebig größeren Zeitspannen, an der jeweiligen Arbeitselektrode zu realisieren.

Wegen der oben beschriebenen Zeitanforderungen ist die Verwendung einer makroskopischen Potentiostatenschaltung gerade für sehr kleine Elektrodenflächen, d.h. für Elektroden mit sehr geringer Oberfläche, nicht möglich, da Verdrahtungskapazitäten und Gerätekapazitäten die erforderliche Signalbandbreite nicht zulassen. Da bei dem im Folgenden beschriebenen erfindungsgemäßen DNA-Analysesystem die Betriebsschaltungen unmittelbar unterhalb der Elektrode in einem Pixel angeordnet sind, reduziert sich die parasitäre Kapazität, die die Arbeitselektrode treiben muss, erheblich und es können sehr kurze Sprungzeiten erreicht werden.

Kurze Sprungzeiten sind erforderlich, da in diesem Fall leicht zwischen unmittelbar auf der Elektrode vorhandenen elektrochemisch aktiven Stoffen und diffusiven Beiträgen zum Oxidationsstrom bzw. zum Reduktionsstrom unterschieden werden kann. Die unmittelbar auf der jeweiligen Elektrode vorhandenen Stoffe werden innerhalb kürzester Zeit oxidiert bzw. reduziert, während die in dem Analyten vorhandenen freien Stoffe aufgrund ihrer spezifischen Diffusionskonstanten wesentlich später zum Signalstrom beitragen.

Bei einem Spannungssprungexperiment teilt sich der Strom an den Arbeitselektroden im Wesentlichen in drei Beiträge auf:
Umladung der Doppelschichtkapazität:Die Doppelschichtkapazität ist immer zu beachten, wenn eine leitfähige Elektrode mit einem Elektrolyten in Kontakt steht. In unmittelbarer Nähe zur Elektrodenoberfläche ordnen sich Ionen in im Wesentlichen zwei Schichten an. Dieses Schichtsystem kann als (Elektrolyt-)Kondensator aufgefasst werden, bei dem die beiden Kondensatorplatten lediglich wenige Moleküle voneinander entfernt sind. Aufgrund dieser Tatsache weist der (Elektrolyt-)Kondensator außerordentlich hohe Werte für die Flächenkapazität auf. Typischerweise werden Werte von 0,1 F/m² erreicht. Bezüglich des erfindungsgemäßen DNA-Sensors zeigt sich, dass die Ladungsmenge, die in die Doppelschichtkapazität fließt, in der gleichen Größenordnung ist wie die Ladungsmenge, die zur Oxidation des Ferrocens aufgebracht wird.
Oxidation des Ferrocens:Das auf der Elektrodenoberfläche vorhandene Ferrocen wird bei einem effektiv positiven Spannungssprung an der Arbeitselektrode unmittelbar oxidiert. Da die hybridisierten Moleküle nur höchstens wenige Nanometer von der Oberfläche entfernt sind, erfolgt die Oxidation dieser Moleküle nahezu vollständig innerhalb der Sprungzeit der Spannung. Messtechnisch überlagert sich dieser Strombeitrag also direkt mit dem Ladestrom der Doppelschichtkapazität.
Oxidation diffundierender Ferrocen-Moleküle:In dem Analyten befindet sich eine nennenswerte Konzentration an Ferrocen-Molekülen, unter anderem jene, die von den Fängermolekülen verdrängt wurden. Diese Moleküle diffundieren aus der Lösung an die Elektrode und führen zu einem elektrischen Stromfluss, der nach dem Spannungsstrom langsam abklingt.

Das primäre Messsignal in diesem Fall ist der Strom, der an der Elektrode während des Spannungssprungs fließt. Die messtechnisch relevante Größe ist jedoch die Ladungsmenge, die zur Stabilisierung der Elektrodenspannung benötigt wird. Diese Ladungsmenge ist ein direktes Maß für die absolute Menge an Ferrocen, welche oxidiert wurde. Vorzugsweise ist für diesen Fall in der Sensor-Anordnung ein Integrator vorgesehen, der den jeweiligen Elektrodenstrom integriert.

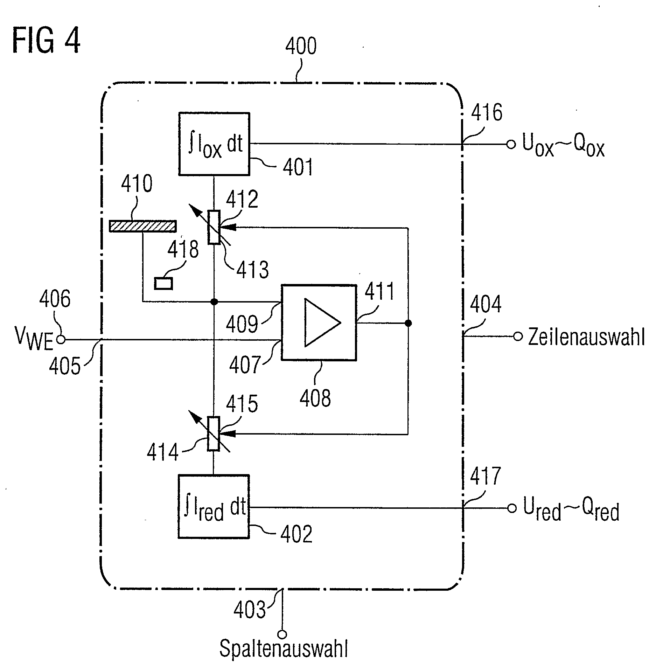

Wie in **Fig.4** in der Pixelschaltung 400 gezeigt ist, ist der jeweilige Integrator 401, 402 direkt in einem jeweiligen Sensorpixel enthalten. Die im Folgenden beschriebenen Pixelschaltungen sind jeweils so zu verstehen, dass jede Pixelschaltung örtlich unterhalb bzw. in unmittelbarer Nähe einer jeweiligen Arbeitselektrode, wie sie in Fig. 1 gezeigt ist, angeordnet und mit dieser elektrisch gekoppelt ist zum Erfassen an der Arbeitselektrode jeweils auftretender Sensorereignisse.

Die Pixelschaltung 400 gemäß Fig.4 weist neben einem Oxidations-Integrator 401 und einem Reduktions-Integrator 402 drei Eingänge 403, 404, 405 sowie zwei Ausgänge 416, 417 auf.

Ein erster Eingang 403, auch bezeichnet als Spaltenauswahl-Eingang ist mit der Spaltensteuerung aus Fig.1 gekoppelt. Über den Spaltenauswahl-Eingang 403 wird der Pixelschaltung 400 das Spaltenauswahl-Signal zugeführt.

Ein zweiter Eingang 404, auch bezeichnet als Zeilenauswahl-Eingang 404, ist mit der in Fig.1 gezeigten Zeilensteuerung gekoppelt und dient zur Zuführung des Zeilenauswahl-Signals an die Pixelschaltung 400.

Ferner ist ein dritter Eingang 405 vorgesehen zum Zuführen eines Referenzpotentials V_{WE} 406 einem ersten Eingang 407 eines ebenfalls in der Pixelschaltung 400 vorgesehenen Operationsverstärkers 408. Der zweite Eingang 409 des Operationsverstärkers 408 ist mit der Arbeitselektrode 410 der jeweiligen Pixelschaltung 400 gekoppelt.

Der Ausgang 411 des Operationsverstärkers 408 ist mit einem Steuereingang 412 zum Verändern eines elektrischen Widerstandes eines ersten regelbaren elektrischen Widerstands 413, dessen einer Anschluss mit dem Eingang des Oxidations-Integrators 401 gekoppelt ist und dessen anderer Anschluss mit dem zweiten Eingang 409 des Operationsverstärkers 408 sowie mit der Arbeitselektrode 410 und mit einem ersten Anschluss eines zweiten regelbaren Widerstands 414 gekoppelt ist, dessen zweiter Anschluss mit dem Eingang des Reduktions-Integrators 402 gekoppelt ist. Der Steueranschluss 415 des zweiten regelbaren Widerstands 414 ist ebenfalls mit dem Ausgang 411 des Operationsverstärkers 408 gekoppelt. Der Ausgang des Oxidations-Integrators 401 ist mit einem ersten Ausgang 416 der Pixelschaltung 400 gekoppelt und der Ausgang des Reduktions-Integrators 402 ist mit einem zweiten Ausgang 417 der Pixelschaltung 400 gekoppelt. Der durch die Arbeitselektrode fließende Elektrodenstrom 418 wird mittels der Integratoren 401 bzw. 402 integriert. Die sich ergebende elektrische Spannung Uₒₓ am Ausgang des Oxidations-Integrators 401 bzw. U_{red} am Ausgang des Reduktions-Integrators 402 ist proportional zu der jeweiligen Oxidations-Ladungsmenge bzw. Reduktions-Ladungsmenge als messtechnisch relevante Größe.

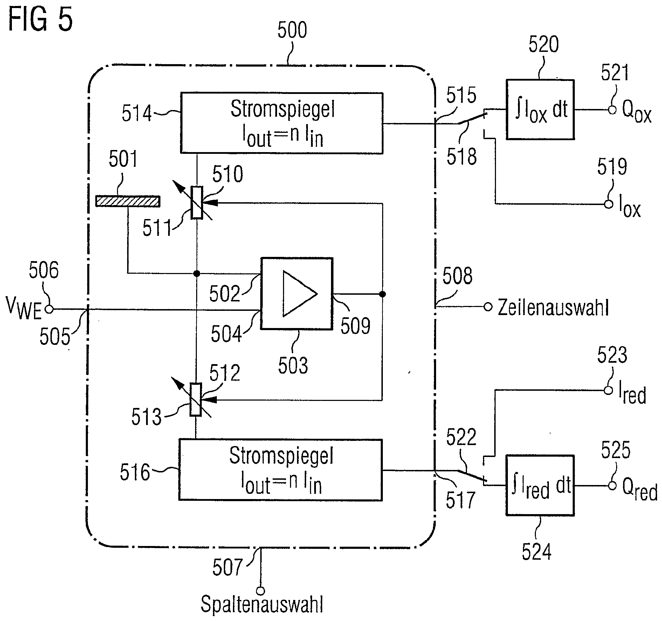

Gemäß einer alternativen Ausgestaltung ist der jeweilige Integrator außerhalb der Pixelschaltung vorgesehen, wie beispielsweise in der Pixelschaltung 500 in **Fig.5** gezeigt.

Auch bei der Pixelschaltung 500 gemäß Fig.5 ist die Arbeitselektrode 501 mit einem ersten Eingang 502 eines Operationsverstärkers 503 gekoppelt, dessen zweiter Eingang 504 mit einem dritten Eingang 505 der Pixelschaltung 500 gekoppelt ist zum Zuführen des Referenzpotentials V_{WE} 506.

Die Pixelschaltung 500 weist ferner einen Spaltenauswahl-Eingang 507 sowie einen Zeilenauswahl-Eingang 508 auf, wobei der Spaltenauswahl-Eingang 507 mit der Spaltensteuerungseinheit und der Zeilenauswahl-Eingang 508 mit der Zeilensteuerungseinheit aus Fig.1 gekoppelt ist.

Der Ausgang 509 des Operationsverstärkers 503 ist mit einem Steueranschluss 510 eines ersten regelbaren Widerstands 511 gekoppelt sowie mit einem Steueranschluss 512 eines zweiten regelbaren Widerstands 513.

Der erste regelbare Widerstand 511 ist mit dessen ersten Anschluss mit einem Eingang einer ersten Stromspiegelschaltung 514 gekoppelt, deren Ausgang mit einem ersten Ausgang 515 der Pixelschaltung 500 gekoppelt ist. Der zweite Anschluss des ersten regelbaren Widerstands 511 ist mit der Arbeitselektrode 501 sowie mit dem zweiten Eingang 502 des Operationsverstärkers 503 gekoppelt sowie mit einem ersten Anschluss des zweiten regelbaren Widerstands 513, dessen zweiter Anschluss mit einem Eingang einer zweiten Stromspiegelschaltung 516 gekoppelt ist, deren Ausgang wiederum mit einem zweiten Ausgang 517 der Pixelschaltung 500 gekoppelt ist.

Der von der ersten Stromspiegelschaltung 514 mit dem Stromspiegelparameter n multiplizierte verstärkte, durch die Arbeitselektrode 501 fließende elektrischer Strom wird als verstärkter Oxidationsstrom an dem ersten Ausgang 515 bereitgestellt und je nach Schalterposition eines ersten Schalters 518 direkt zur Weiterverarbeitung als erstes StromAusgangssignal Iₒₓ 519 bereitgestellt oder einem Oxidations-Integrator 520 zugeführt, welcher aus dem verstärkten Oxidationsstrom die Oxidations-Spannung Uₒₓ 521 bildet, welche proportional ist zu der Oxidations-Ladungsmenge Qₒₓ.

An dem zweiten Ausgang 517 wird ein verstärkter Reduktionsstrom in entsprechender Weise, multipliziert mit dem Faktor n der zweiten Stromspiegelschaltung 516, bereitgestellt und je nach Schalterposition eines zweiten Schalters 522 direkt als Reduktions-Ausgangs-Strom I_{red} 523 oder einem Reduktions-Integrator 524, welcher eine Reduktions-Ausgangsspannung U_{red} 525 bereitstellt, welche proportional ist zu der Reduktions-Ladungsmenge Q_{red}.

In diesem Fall wird der jeweils aktive ausgewählte Sensor, d.h. die jeweilige ausgewählte Pixelschaltung 500 mittels einer Auswahllogik (in Fig.5 repräsentiert durch die symbolischen Schalter 518 und 522) mit dem jeweiligen zentralen Integrator (Oxidations-Integrator 520 bzw. Reduktions-Integrator 524) verbunden und der entsprechend fließende Strom wird gemessen.

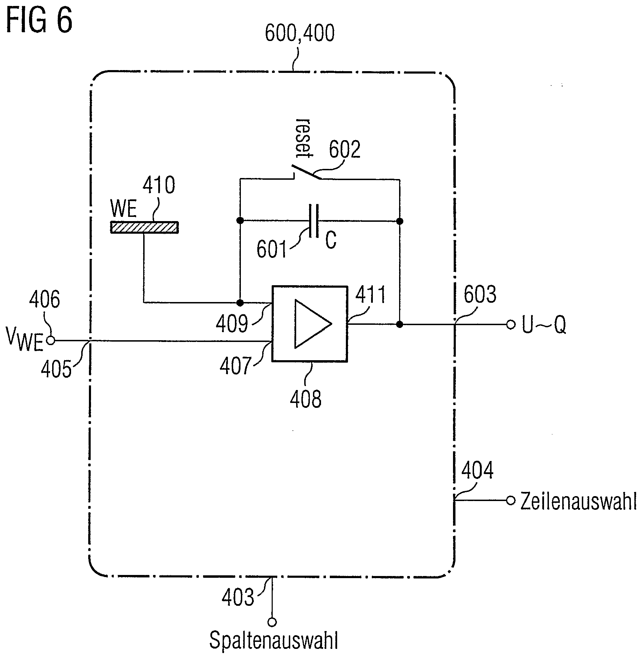

Ist der Integrator in der Pixelschaltung enthalten, wie beispielsweise in der Pixelschaltung 600 in **Fig.6** dargestellt, so arbeiten die Pixelschaltungen 600 autark und es können prinzipiell alle Sensoren eines Arrays auf einmal ausgewertet werden.

Die Abfrage der Integratorergebnisse kann nach dem jeweiligen Spannungssprungexperiment sukzessive mittels einer geeigneten Auswahllogik erfolgen.

Fig.6 zeigt in vereinfachter Darstellung die Pixelschaltung 400 gemäß Fig.4, wobei lediglich dargestellt ist, dass der erste Eingang 407 des Operationsverstärkers 408 mit dem dritten Eingang 405 der Pixelschaltung 400 gekoppelt ist sowie die Arbeitselektrode mit dem zweiten Eingang 409 des Operationsverstärkers 408.

Ferner ist ein Rückkoppelkondensator 601 dargestellt, dessen erster Anschluss mit dem Ausgang 411 des Operationsverstärkers 408 gekoppelt ist und dessen zweiter Anschluss rückgekoppelt ist zu dem zweiten Eingang 409 des Operationsverstärkers 408. Ein Rücksetzschalter 602 ist parallel geschaltet zu dem Rückkoppelkondensator 601 und gekoppelt mit einerseits dem zweiten Eingang 409 des Operationsverstärkers 408 sowie andererseits dem Ausgang 411 des Operationsverstärkers 408, welcher mit dem Ausgang 603 der Pixelschaltung 600 gekoppelt ist und an dem das zu der Ladungsmenge proportionale Ausgangs-Spannungssignal bereitgestellt wird.

Insbesondere kann die Arbeitselektrodenschaltung derart eingerichtet sein, dass die Ladungsmenge aus der Doppelschichtkapazität, die keine Information aufgrund einer Funktionalisierung trägt, kompensiert werden kann. In diesem Fall ist in dem Sensorarray eine Elektrode vorhanden, die nicht funktionalisiert ist, d.h. auf der keine Fängermoleküle immobilisiert sind und die somit ausschließlich die Ladung aus der Doppelschichtkapazität und den diffusiven Anteil misst.

Der elektrische Strom, der durch diese zusätzliche Elektrode während des Spannungssprungexperiments fließt oder die integrierte elektrische Ladung kann jeweils von den Signalen der aktiven Sensoren abgezogen werden, so dass diese Pixelschaltungen nur noch das messtechnisch relevante Ladungssignal bzw. Stromsignal liefern.

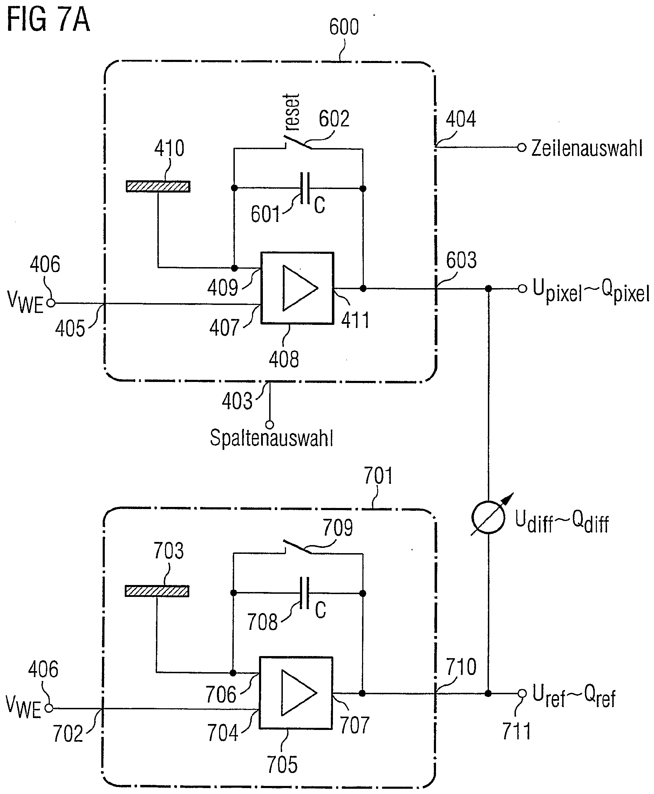

In **Fig.7a** ist ein solches Ausführungsbeispiel gezeigt, wobei die Pixelschaltung gemäß der in Fig.6 gezeigten Ausführungsform ausgebildet ist.

Wie in Fig.7a dargestellt ist, ist gemäß dieser Ausführungsform eine Referenzschaltung 701 vorgesehen mit einem Eingang 702, an dem das Referenzpotential V_{WE} bereitgestellt ist. Ferner ist eine Referenz-Arbeitselektrode 703 vorgesehen, auf der keine Fängermoleküle immobilisiert sind bzw. die mit einer nicht bindungsbereiten Beschichtung versehen ist.

Der Eingang 702 der Referenzschaltung 701 ist mit einem ersten Eingang 704 eines Referenz-Operationsverstärkers 705 gekoppelt, dessen zweiter Eingang 706 mit der Referenz-Arbeitelektrode 703 gekoppelt ist. Ein Ausgang 707 des Referenz-Operationsverstärkers ist über einen Referenz-Kondensator 708 an den zweiten Eingang 706 des Referenz-Operationsverstärkers 705 rückgekoppelt sowie gegebenenfalls über einen Referenz-Rücksetzschalter 709 zum Rücksetzen des Integrators mit dem zweiten Eingang 706 des Referenz-Operationsverstärkers 705 kurzgeschlossen. Ein Ausgang 710 der Referenzschaltung 701 liefert das integrierte Referenzsignal U_{ref} 711. Als Messsignal für die elektrochemischen Umsetzungen an der Arbeitselektrode 410 des jeweiligen Pixels wird die Differenz zwischen den Ausgangsspannungen der beiden Integratoren, d.h. die Differenz der von der Pixelschaltung 600 bereitgestellten Ausgangsspannung und der Referenz-Ausgangsspannung, welche an dem Ausgang 710 der Referenzschaltung bereitgestellt wird, herangezogen.

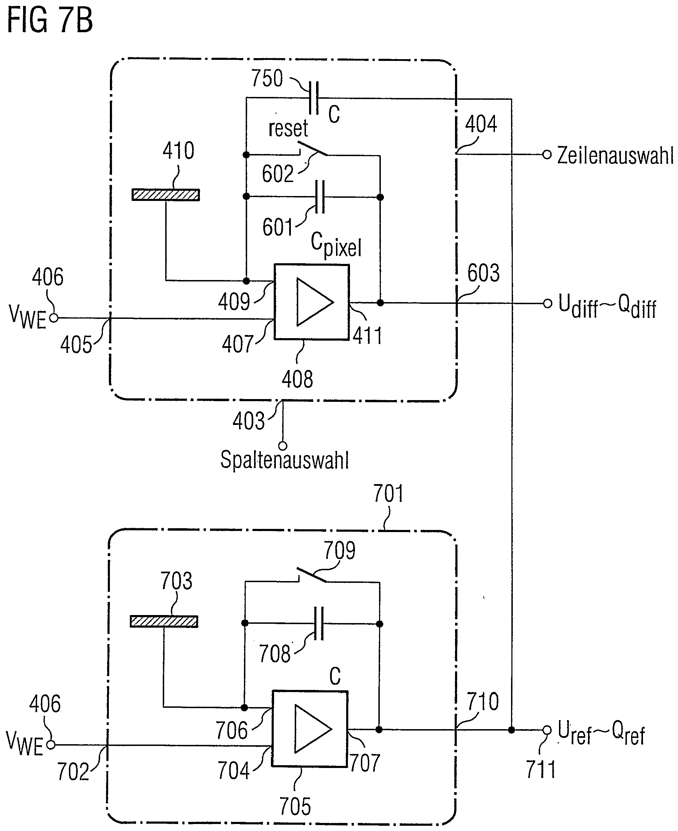

Die in **Fig.7b** gezeigte Ausführungsform unterscheidet sich von der in Fig.7a gezeigten Ausführungsform dadurch, dass ein zentrales Referenzpixel vorgesehen ist, welches ein Referenz-Spannungssignal generiert, das über einen Koppelkondensator 750, welcher geschaltet ist zwischen den Ausgang der zentralen Referenzschaltung 701, welche den gleichen Aufbau hat wie die Referenzschaltung 701 gemäß Fig.7a, welche in jedem Pixel vorgesehen ist, und dem zweiten Eingang 409 des jeweiligen Operationsverstärkers 408 in der Pixelschaltung 600. Das in diesem Fall zentrale Referenz-Spannungssignal U_{ref} 711 wird somit über den Koppelkondensator 750 auf alle aktiven Sensorelektroden zurückgekoppelt und die Doppelschicht-Kapazität und die diffusiven Umsetzungen werden exakt eliminiert.

Das Ausgangssignal der jeweiligen Pixelschaltung 600 gibt daher direkt nur noch das Signal der elektrochemischen Umsetzungen aufgrund der Funktionalisierung der Elektroden aus. Dies ermöglicht eine sehr empfindliche Messung der an dem jeweiligen Sensor, d.h. an der Arbeitselektrode auftretenden Ladungsmengen.

Treten die elektrischen Ladungen, die aus den Oxidationsvorgängen oder den Reduktionsvorgängen stammen, zeitlich deutlich später auf als die Dauer des Spannungssprungs, so können die elektrischen Ladungsträger aus dem Laden der Doppelschichtkapazität auf das verspätete Aktivieren des Integrators ausgeblendet werden.

Der jeweilige Integrator wird also erst eingeschaltet in diesem Fall bzw. freigegeben, wenn die Spannung an der Arbeitelektrode bzw. an den Arbeitselektroden den Zielwert erreicht hat und die messtechnisch relevanten elektrochemischen Umsetzungen erfolgen.

Wie oben beschrieben wurde, hat die Betriebsschaltung der Arbeitselektroden die Aufgabe, das Elektrodenpotential konstant zu halten und den durch die Arbeitselektroden fließenden Strom und/oder die Ladung bei einem oxidativen Spannungsstrom und/oder reduktiven Spannungsstrom zu messen. Hierzu ist in der Pixelschaltung eine Differenzstufe vorgesehen, die das Elektrodenpotential mit einem Sollpotential vergleicht und dem zum Halten der elektrischen Spannung notwendigen Strom misst und/oder die Ladungsmenge speichert. Da der elektrische Strom aufgrund des schnellen Spannungssprungs bei großen/kleinen Elektroden sehr große/kleine Werte annehmen kann, ist es zweckmäßig, den Elektrodenstrom vor einer Weiterverarbeitung mittels Stromspiegelschaltungen derart zu verstärken bzw. zu teilen, dass der Strom für die vorzugsweise eingesetzte integrierte CMOS-Schaltungstechnik übliche und mittels üblicher digitaler Schaltungen weiterverarbeitbare Werte annimmt.

Die in den Ausführungsformen beschriebenen Stromspiegel weisen daher den Teilungsfaktor/Verstärkungsfaktor n auf. Der Ausgangsstrom der Stromspiegelschaltungen beträgt also das n-fache des Eingangsstroms der Stromspiegelschaltungen. n kann kleiner als 1 sein (beispielsweise 1/100, 1/10 für vergleichsweise große Elektroden) oder größer als 1 sein (10, 100 für vergleichsweise kleine Elektroden).

Zur Messung oxidativer Signale sowie reduktiver Signale sind zwei komplementäre Strompfade notwendig, die den jeweiligen Elektrodenstrom aufnehmen bzw. liefern können, wie beispielsweise in der Pixelschaltung 500 in Fig.5 gezeigt.

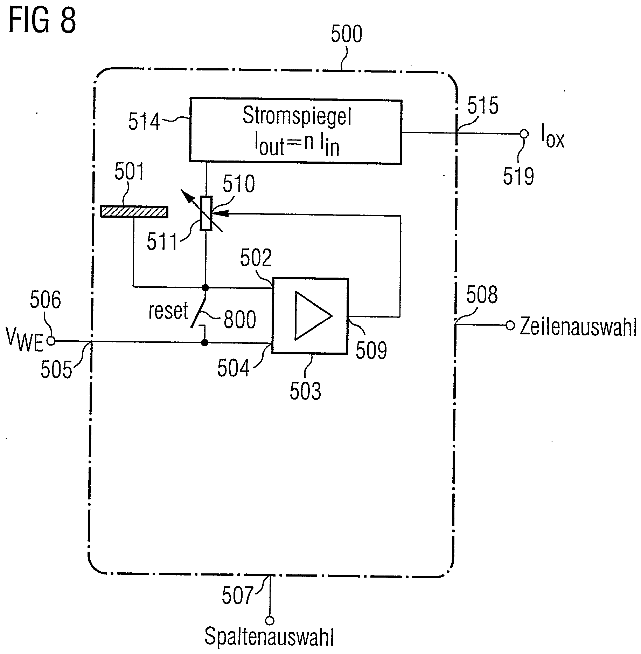

Üblicherweise ist jedoch lediglich eines der beiden elektrochemischen Signale (Oxidation/Reduktion) messtechnisch relevant, weshalb lediglich nur ein Zweig, in vereinfachter Darstellung beispielsweise in Fig.6 gezeigt, vorgesehen ist. Zur Vereinfachung und Miniaturisierung der Pixelschaltungen kann daher einer der Stromzweige in einer Pixelschaltung durch einen einfachen Schalter 800 (vgl. **Fig.8**) ausgeführt sein, der vor der eigentlichen Messung die Arbeitselektrode 501 mit dem Sollpotential, d.h. den dritten Eingang 505 der Pixelschaltung 500 verbindet und einen eventuell fließenden elektrischen Strom an diese Spannungsquelle abführt.

Es ist ferner wünschenswert, dass die Betriebsschaltung der Arbeitselektroden einen sehr hohen Dynamikbereich für den Elektrodenstrom aufweist.

Während des Spannungssprungs treten kurzzeitig sehr große elektrische Ströme auf (Doppelschichtkapazität, Oxidationssignal). Sowohl dieser große Impulsstrom als auch der anschließende kleine Strom aus weiteren Oxidationsprozessen müssen von der Betriebsschaltung der Arbeitselektroden korrekt gemessen werden.

Aus diesem Grund sind sehr präzise Stromspiegelschaltungen wünschenswert. Eine hohe Genauigkeit der Stromspiegelschaltungen kann durch großflächige Stromspiegelschaltungen erreicht werden, damit prozesstechnisch bedingte statistische Schwankungen der Bauteilparameter der Stromspiegelschaltungen einen bestimmten Wert nicht überschreiten. Da großflächige Transistoren, die eine Stromspiegelschaltung bilden, auch eine große parasitäre Kapazität aufweisen, wäre die Genauigkeit der in der Pixelschaltung integrierten Stromspiegelschaltung begrenzt, da diese Kapazität ebenfalls von dem Elektrodenstrom aufgeladen werden muss. Die Ladungsmenge erscheint nicht oder erst sehr spät an dem Ausgang der Stromspiegelschaltung und steht somit nicht für die Integration zur Verfügung.

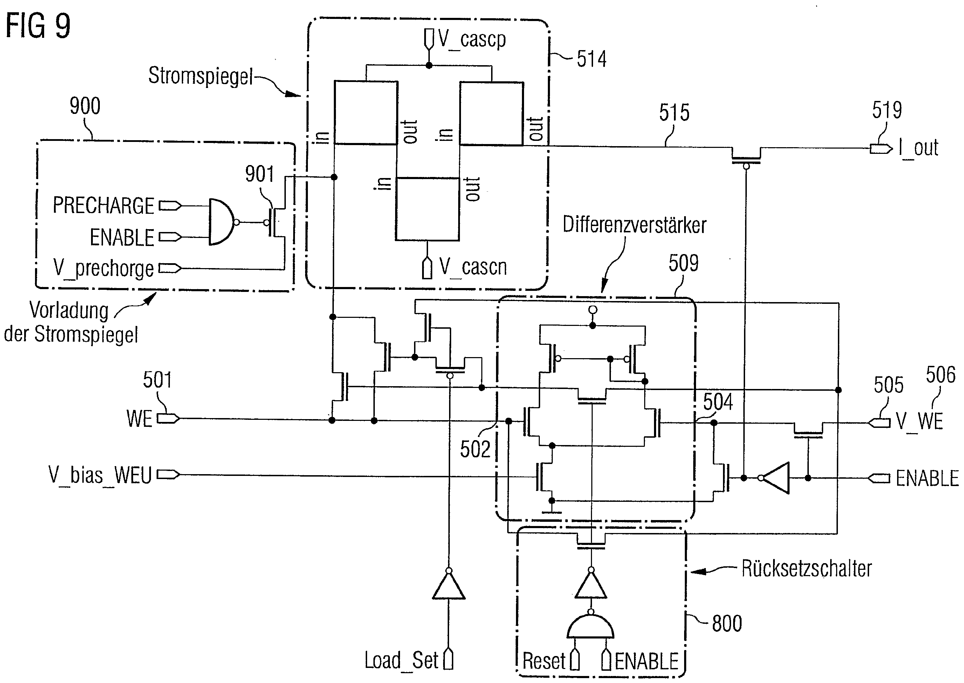

Für eine hohe Genauigkeit und Messgeschwindigkeit ist es zweckmäßig, die Stromspiegeleinheiten in der Sensor-Pixelschaltung vor dem Spannungssprung vorzuladen. Dies kann mittels eines Schalttransistors 901 einer Vorladeeinheit 900 (vgl. **Fig.9**), welche mit der ersten Stromspiegelschaltung 514 gekoppelt ist, erfolgen, wobei der Schalttransistor 901 einen geeigneten Strom in die erste Stromspiegelschaltung 514 einprägt und kurz vor dem Spannungssprung wieder ausgeschaltet wird.

In einer optionalen Ausführungsform können, wie in Fig.9 dargestellt, zusätzliche Schaltelemente vorgesehen sein, welche zum Einstellen der Flankensteilheit vorteilhaft sind.

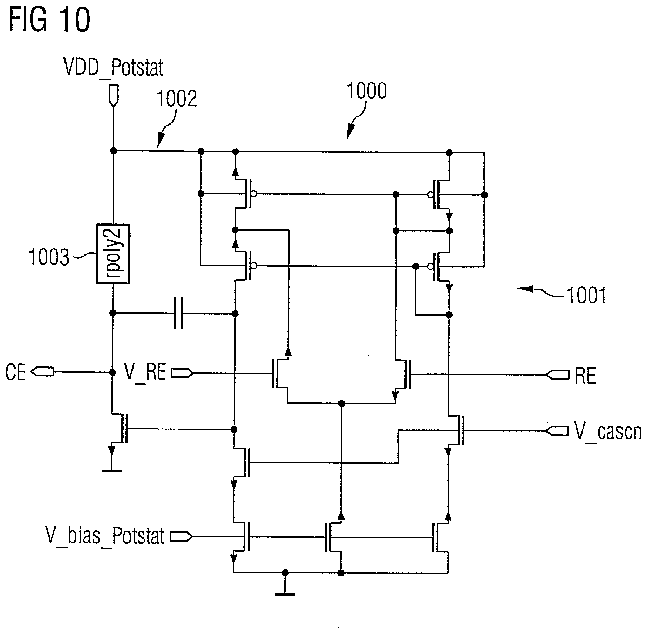

Die in **Fig.10** dargestellte Potentiostatenschaltung 1000 dient zur Kontrolle des Elektrolytpotentials.

Hierzu ist im Reaktionsvolumen eine Referenzelektrode angebracht, die das Elektrolytpotential misst, mit einer Referenzspannung vergleicht und über eine großflächige Gegenelektrode des Elektrolytpotentials geeignet einstellt.

Da die Potentiostatenschaltung 1000 in erfindungsgemäßen Messverfahren das Potential des Elektrolyten sprunghaft ändern muss, weist diese erfindungsgemäß eine hohe Signalbandbreite sowie eine hohe Stromtreibfähigkeit auf.

Da das elektrochemische System Referenzelektrode/Elektrolyt/Gegenelektrode komplexe und zudem von den Inhaltstoffen des Elektrolyten abhängige elektrische Eigenschaften aufweist, ist es notwendig, die Potentiostatenschaltung 1000 zur Vermeidung von Oszillationen elektrisch sehr stabil auszuführen.

Dies wurde gemäß dieser Ausgestaltung des Aspekts der Erfindung dadurch gelöst, dass ein zweistufiger Operationsverstärker 1001 verwendet wird, der im positiven Ausgangszweig 1002 einen ohmschen Widerstand 1003 aufweist. Dies reduziert zwar einerseits die Verstärkung des Operationsverstärkers 1001, erlaubt jedoch einen stabilen Betrieb bis zu hohen Frequenzen bei gleichzeitig hohen Ausgangsströmen für kapazitive Lasten.

Um eine steile Flanke beim Spannungssprung zu erreichen, wird die analoge Sprungspannung nicht von außerhalb des elektronischen Chips bereitgestellt, sondern die beiden elektrischen Potentiale liegen als stabiles Gleichspannungssignal an dem elektronischen Chip an. Nach einem Trigger-Impuls wird die Eingangsspannung an der Potentiostatenschaltung 1000 zwischen den beiden Gleichspannungssignal-Werten umgeschaltet. Die Umschaltung kann sehr schnell erfolgen und benötigt lediglich ein TTL-Signal (**T**ransistor-**T**ransistor-**L**ogik-Signal).

Werden die erfindungsgemäßen Sensoren in einem Array angeordnet, so werden die einzelnen Sensoren mittels Zeilenleitungen und Spaltenleitungen adressiert, wie beispielsweise in Fig.1 für eine Sensor-Anordnung gezeigt ist. Somit können die einzelnen Sensoren selektiv angesteuert und/oder ausgelesen werden.

Im Zusammenhang mit den oben beschriebenen Messprinzipien können alle Verschaltungen der Sensorpixel eingesetzt werden, wie sie beispielsweise im Zusammenhang mit Redox-Cycling-Sensoren an sich bekannt sind.

Für den Fall, dass in einem jeweiligen Sensorpixel kein Integrator vorgesehen ist, wird der im aktiven Pixel gemessene und konditionierte elektrische Strom über Zeilenleitungen oder Spaltenleitungen an den Rand des Sensorarrays geleitet und kann dort nach weiterer Signalverarbeitung in analoger Signalform oder in digitaler Signalform aus dem elektronischen Chip geleitet werden oder analog oder digital integriert werden.

Das Sensorarray wird auf diese Weise sukzessive ausgelesen. Da eine einzelne Messung lediglich eine Zeit in der Größenordnung einer von 1 ms benötigt, kann das gesamte Sensorarray vergleichsweise zügig ausgelesen werden. Insbesondere können mehrere Sensorpixel gleichzeitig aktiviert werden, wenn die entsprechende Anzahl von Komparatoren vorhanden ist.

Vorzugsweise wird eine gesamte Spalte oder Zeile aktiviert und das Sensorsignal am Rande der Matrix erfasst. Bei allen inaktiven Sensorpixeln wird die Elektrode stromlos geschaltet bzw. deren Spannung gegenüber dem Elektrolytpotential derart gewählt, dass an dieser Elektrode keine unerwünschten elektrochemischen Umsetzungen stattfinden.

Ist in den Sensorpixeln jeweils ein Integrator enthalten, so können im Prinzip alle Sensorpixel gleichzeitig aktiviert werden und in einem folgenden Zeitintervall die Integrationsergebnisse sukzessive mittels Auswahlleitungen von den einzelnen Pixeln abgefragt werden.

In der Peripherie der Sensormatrix befinden sich weitere elektronische Schaltungen zum elektrochemischen Betrieb des Sensors wie beispielsweise die Potentiostatenschaltungen sowie Auswerteschaltungen für die analogen Messsignale und/oder digitalen Messsignale, beispielsweise Analog-/Digital-Wandler sowie ferner digitale Schaltungen und analoge Schaltungen zur Ansteuerung des Sensorarrays.

Besonders vorteilhaft ist eine vollständig digitale Kommunikation zwischen dem erfindungsgemäßen elektrochemischen Analysesystem, d.h. Sensor-Anordnung und einem peripheren Lesegerät.

In diesem Fall ist insbesondere zusätzlich ein Analog-/Digital-Wandler zur Umwandlung des analogen Messsignals in ein digitales Datensignal sowie ein Digital-/Analog-Wandler zur Erzeugung der benötigten Elektrodenspannung in der Sensor-Anordnung vorgesehen und in dieser monolithisch integriert. Ferner ist in diesem Fall eine Recheneinheit zur digitalen Kommunikation mit dem Lesegerät sowie eine Kommunikations-Schnittstelle an der Sensor-Anordnung vorgesehen zur bidirektionalen Datenkommunikation mit dem Lesegerät.

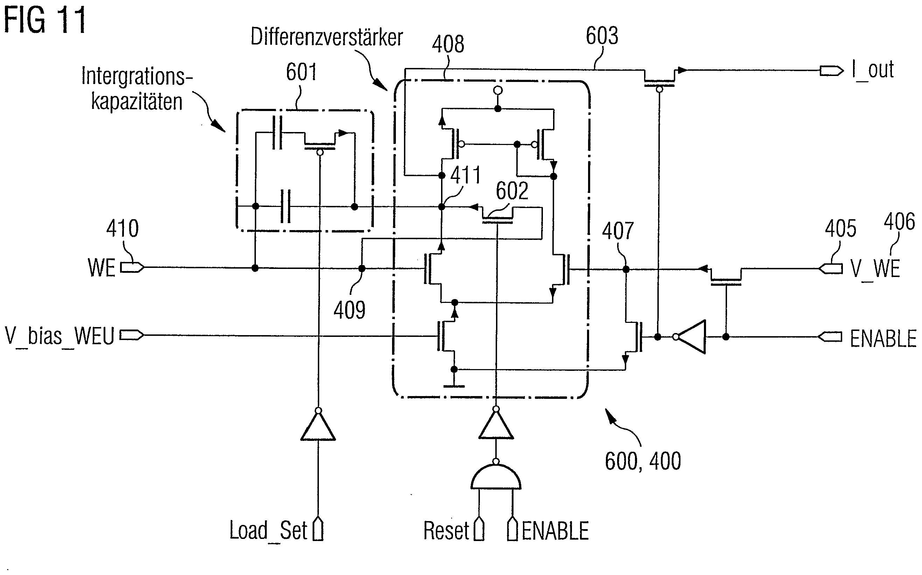

**Fig.11** zeigt eine schaltungstechnische Realisierung der Pixelschaltung 600 gemäß Fig.6.

In diesem Dokument sind folgende Veröffentlichungen zitiert:
[1] Hofmann, F. et al., "Passive DNA Sensor with Gold Electrodes Fabricated in a CMOS Backend Process", Proc. ESSDERC 2002, Digest of Techn. Papers, Seiten 487-490.
[2] Thewes, R. et al., "Sensor Arrays for Fully Electronic DNA Detection on CMOS", ISSC 2002, Digest of Techn. Papers, Seiten 350-351.
[3] Hintsche, R. et al., "Microelectrode Arrays and Application to Biosensing Devices", Biosensors and Bioelectronics, 1994, Vol. 9, Seiten 697-705.
[4] Hintsche, R. et al., "Microbiosensors Using Electrodes Made in Si-Technology", Frontiers in Biosensorics, Fundamental Aspects, 1997, F.W. Scheller et al. (eds.), Dirk Hauser Verlag, Basel, Seiten 267-283.
[5] Paeschke, M. et al., "Voltametric Multichannel Measurements Using Silicon Fabricated Microelectrode Arrays", Electroanalysis, 1996, Vol. 7, No. 1, Seiten 1-8.
[6] Erhältlich im Internet am 31.3.2004 unter der URL-Adresse:http://www.combimatrix.com
[7] Erhältlich im Internet am 31.3.2004 unter der URL-Adresse:http://www.frizbiochem.com

## Patentansprüche

1. Sensor-Anordnung, die ein Sensor-Array und ein Substrat aufweist, wobei das Sensor-Array monolithisch in und/oder auf dem Substrat integriert ist, mit mindestens drei Elektroden, wobei die Mehrzahl der mindestens drei Elektroden als Biosensor-Anordnungen zum Erfassen von Biomolekülen eingerichtet sind, und einer Schalteinheit, die derart eingerichtet ist, dass mittels der Schalteinheit wahlweise mindestens eine der mindestens drei Elektroden als mit einer Elektrolytlösung eines elektrolytischen Analyten gekoppelte Arbeitselektrode (WE, 210) schaltbar ist und dass mindestens zwei der Elektroden der mindestens drei Elektroden wahlweise als Gegenelektrode (CE) schaltbar sind und wobei die mindestens drei Elektroden derart eingerichtet sind, dass an einer als Arbeitselektrode (WE, 210) geschalteten Elektrode der mindestens drei Elektroden in der Elektrolytlösung bei Anwesenheit des elektrolytischen Analyten Sensorereignisse stattfinden, **gekennzeichnet durch** einen Steuerschaltkreis, der zum Ansteuern, Auswählen und/oder Auslesen der mindestens drei Elektroden oder eines Teils der mindestens drei Elektroden eingerichtet ist, die Gegenelektrode (CE) durch Zusammenschalten von als Arbeitselektroden unbenutzten Elektroden des Sensor-Arrays zu realisieren.

2. Sensor-Anordnung nach Anspruch 1, wobei die Schalteinheit mindestens einen MOS-Transistor umfasst, der innerhalb des Sensor-Arrays verwirklicht ist.

3. Sensor-Anordnung nach Anspruch 1, wobei die Sensor-Anordnung mindestens eine Betriebsschaltung aufweist und die Schalteinheit außerhalb des Sensor-Arrays verwirklicht ist und die mindestens eine Betriebsschaltung (208) der als gemeinsame Gegenelektrode (CE) des Sensor-Arrays geschalteten mindestens zwei Elektroden mittels der Schalteinheit derart ansteuerbar ist, dass das Elektrodenpotential dem Potential am Gegenelektrodenanschluß eines Potentiostaten folgt.

4. Sensor-Anordnung nach einem der Ansprüche 1 bis 3, mit 50 bis 1000000, bevorzugt mit 100 bis 250000 Elektroden.

5. Sensor-Anordnung nach einem der Ansprüche 1 bis 4, wobei die Mehrzahl der mindestens drei Elektroden zum Erfassen von oxidierbaren bzw. reduzierbaren Stoffen eingerichtet ist.

6. Sensor-Anordnung nach einem der Ansprüche 1 bis 5, eingerichtet zum Erfassen von Nukleinsäuremolekülen, Peptiden und/oder Proteinen.

7. Sensor-Anordnung nach einem der Ansprüche 1-6, wobei auf der Oberfläche der Elektroden Fängermoleküle immobilisiert sind.

8. Sensor-Anordnung nach einem der Ansprüche 1-7,
das derart eingerichtet ist, dass in Anwesenheit eines elektrolytischen Analyten mittels elektrochemisch aktiver Markierungen ein Sensorsignal generierbar ist.

9. Sensor-Anordnung nach Anspruch 8, wobei die elektrochemisch aktiven Markierungen Metallkomplex-Markierungen aufweisen.

10. Sensor-Anordnung nach Anspruch 8 oder 9, wobei die elektrochemisch aktiven Markierungen Ferrocen-Markierungen aufweisen.

11. Verfahren zum Betreiben einer Sensor-Anordnung, die ein Sensor-Array und ein Substrat aufweist, wobei das Sensor-Array in und/oder auf dem Substrat monolithisch integriert ist, in einem elektrochemischen Analyseverfahren, wobei die Sensor-Anordnung mindestens drei Elektroden, deren Mehrzahl als Biosensor-Anordnungen zum Erfassen von Biomolekülen eingerichtet sind, und eine Schalteinheit aufweist, die derart eingerichtet ist, dass mittels der Schalteinheit wahlweise mindestens eine der mindestens drei Elektroden als mit einer Elektrolytlösung eines elektrolytischen Analyten gekoppelte Arbeitselektrode (WE, 210) schaltbar ist und mindestens zwei der Elektroden wahlweise als Gegenelektrode (CE) schaltbar sind und wobei die mindestens drei Elektroden derart eingerichtet sind, dass an einer als Arbeitselektrode (WE, 210) geschalteten Elektrode der mindestens drei Elektroden in der Elektrolytlösung bei Anwesenheit des elektrolytischen Analyten Sensorereignisse stattfinden, wobei gemäß dem Verfahren
(a) eine erste Anzahl der mindestens drei Elektroden, die aus mindestens zwei Elektroden besteht, zusammengeschaltet wird, mit dem Gegenelektrodenanschluß eines Potentiostaten verbunden wird und hierdurch die Gegenelektrode des Sensor-Arrays realisiert wird;
(b) eine zweite Anzahl der mindestens drei Elektroden, die aus mindestens einer Elektrode besteht, als Arbeitselektroden (WE, 210) zur Detektion eines elektrolytischen Analyten geschaltet werden;
(c) nach Erfassung der Sensorereignisse an den Arbeitselektroden (WE, 210) in (b) mindestens eine Elektrode der ersten Anzahl der mindestens drei Elektroden als Arbeitselektrode (WE, 210) zur Detektion eines elektrolytischen Analyten geschaltet wird;
(d) mindestens eine Elektrode der zweiten Anzahl der mindestens drei Elektroden gemäß (a) als Gegenelektrode (CE) geschaltet wird; und
(e) die Schritte (a) bis (d) beliebig oft wiederholt werden können.

12. Verfahren zum Betreiben einer Sensor-Anordnung in einem elektrochemischen Analyseverfahren nach Anspruch 11, wobei die Gesamtoberfläche der als Gegenelektroden (CE) geschalteten Elektroden in den Schritten (a) und (d) mindestens 10fach so groß ist, wie die Gesamtoberfläche der als Arbeitselektroden (WE, 210) geschalteten Elektroden.

13. Verfahren zum Betreiben einer Sensor-Anordnung in einem elektrochemischen Analyseverfahren nach Anspruch 11 oder 12, wobei mindestens ein Stoff in einem Elektrolyten qualitativ und/oder quantitative aufgrund dessen charakteristischer Oxydations- bzw. Reduktionsspannung mittels Voltametrie, Amperometrie und/oder Coulometrie bestimmt wird.

14. Verfahren zum Betreiben einer Sensor-Anordnung in einem elektrochemischen Analyseverfahren nach einem der Ansprüche 11 bis 13, wobei zur Analyse von Biomolekülen auf den Elektroden Ferrocen-markierte Fängermoleküle immobilisiert sind und die Konzentration der Biomoleküle durch Messen der bei der Oxydation des Ferrocens fließenden Ladungsmenge bestimmt wird.

15. Verfahren zum Betreiben einer Sensor-Anordnung in einem elektrochemischen Analyseverfahren nach einem der Ansprüche 11 bis 14, wobei zusätzlich eine externe Gegenelektrode zur Verbesserung des Zeitverhaltens der Sensor-Anordnung verwendet wird.

16. Verfahren zum Betreiben einer Sensor-Anordnung in einem elektrochemischen Analyseverfahren nach einem der Ansprüche 11 bis 15, wobei die Sensor-Anordnung mindestens eine Betriebsschaltung zum Ansteuern der Elektroden aufweist und die Betriebsschaltung (208) einer Arbeitselektrode (WE, 210) zur Schaltung als Gegenelektrode (CE) mittels der Schalteinheit deaktiviert wird.

## Claims

1. Sensor arrangement which has a sensor array and a substrate, wherein the sensor array is monolithically integrated in and/or on the substrate, having at least three electrodes, wherein the plurality of the at least three electrodes are designed as biosensor arrangements for detection of biomolecules, and having a switching unit, which is designed in such a manner that, by means of the switching unit, at least one of the at least three electrodes can be selectively connected as a working electrode (WE, 210) which is coupled to an electrolyte solution of an electrolytic analyte and that at least two of the electrodes of the at least three electrodes can be selectively connected as an opposing electrode (CE), and wherein the at least three electrodes are designed in such a manner that sensor events take place at one electrode, which is connected as a working electrode (WE, 210), of the at least three electrodes in the electrolyte solution when in the presence of the electrolytic analyte, **characterized by** a control circuit which is designed to drive, select and/or read the at least three electrodes or some of the at least three electrodes, to provide the opposing electrode (CE) by interconnection of electrodes of the sensor array which are not used as working electrodes.

2. Sensor arrangement according to Claim 1, wherein the switching unit has at least one MOS transistor, which is provided within the sensor array.

3. Sensor arrangement according to Claim 1, wherein the sensor arrangement has at least one operating circuit and the switching unit is provided outside the sensor array, and the at least one operating circuit (208) for the at least two electrodes which are connected as a common opposing electrode (CE) in the sensor array can be driven by means of the switching unit in such a manner that the electrode potential follows the potential at the opposing electrode connection of a potentiostat.

4. Sensor arrangement according to one of Claims 1 to 3, having 50 to 1 000 000, preferably having 100 to 250 000, electrodes.

5. Sensor arrangement according to one of Claims 1 to 4, wherein the plurality of at least three electrodes are designed for detection of substances which can be oxidized or reduced.

6. Sensor arrangement according to one of Claims 1 to 5, designed for detection of nucleic acid molecules, peptides and/or proteins.

7. Sensor arrangement according to one of Claims 1-6, wherein capture molecules are immobilized on the surface of the electrodes.

8. Sensor arrangement according to one of Claims 1 to 7, which is designed in such a manner that a sensor signal can be generated by means of electrochemically active markings in the presence of an electrolytic analyte.

9. Sensor arrangement according to Claim 8, wherein the electrochemically active markings have metal complex markings.

10. Sensor arrangement according to Claim 8 or 9, wherein the electrochemically active markings have ferrocene markings.

11. Method for operation of a sensor arrangement which has a sensor array and a substrate, wherein the sensor array is monolithically integrated in and/or on the substrate in an electrochemical analysis method, wherein the sensor arrangement has at least three electrodes, the majority of which are designed as biosensor arrangements for detection of biomolecules, and has a switching unit which is designed in such a manner that, by means of the switching unit, at least one of the at least three electrodes can be selectively connected as a working electrode (WE, 210) which is coupled to an electrolyte solution of an electrolytic analyte and at least two of the electrodes can be selectively connected as an opposing electrode (CE), and wherein the at least three electrodes are designed in such a manner that sensor events take place at one electrode, which is connected as a working electrode (WE, 210), of the at least three electrodes in the electrolyte solution when in the presence of the electrolytic analyte, wherein, according to the method:
(a) a first number of the at least three electrodes, which number comprises at least two electrodes, are interconnected, are connected to the opposing electrode connection of a potentiostat, thus providing the opposing electrode of the sensor array;
(b) a second number of the at least three electrodes, which number comprises at least one electrode, is or are connected as working electrodes (WE, 210) for detection of an electrolytic analyte;
(c) after detection of the sensor events at the working electrodes in (b), at least one electrode of the first number of the at least three electrodes is or are connected as a working electrode (WE, 210) for detection of an electrolytic analyte;
(d) at least one electrode of the second number of the at least three electrodes according to (a) is or are connected as an opposing electrode (CE); and
(e) steps (a) to (d) can be repeated as often as required.

12. Method for operation of a sensor arrangement in an electrochemical analysis method according to Claim 11, wherein the total surface area of the electrodes which are connected as opposing electrodes (CE) in steps (a) and (d) is at least ten times as large as the total surface area of the electrodes which are connected as working electrodes (WE, 210).

13. Method for operation of a sensor arrangement in an electrochemical analysis method according to Claim 11 or 12, in which at least one substance in an electrolyte is determined qualitatively and/or quantitatively on the basis of its characteristic oxidation or reduction voltage by means of voltammetry, amperometry and/or coulometry.

14. Method for operation of a sensor arrangement in an electrochemical analysis method according to one of Claims 11 to 13, wherein ferrocene-marked capture molecules are immobilized on the electrodes in order to analyze biomolecules, and the concentration of the biomolecules is determined by measurement of the amount of charge flowing during oxidation of the ferrocene.

15. Method for operation of a sensor arrangement in an electrochemical analysis method according to one of Claims 11 to 14, wherein an external opposing electrode is additionally used in order to improve the time response of the sensor arrangement.

16. Method for operation of a sensor arrangement in an electrochemical analysis method according to one of Claims 11 to 15, wherein the sensor arrangement has at least one operating circuit for driving the electrodes and the operating circuit (208) of a working electrode (WE, 210) is deactivated by means of the switching unit, for connection as an opposing electrode (CE).

## Revendications

1. Ensemble de capteurs qui comprend un réseau de capteurs et un substrat, dans lequel le réseau de capteurs est intégré monolithiquement dans et/ou sur le substrat, avec au moins trois électrodes, dans lequel la pluralité des au moins trois électrodes sont conçues sous la forme d'ensembles de biocapteurs pour la détection de biomolécules, et une unité de commutation qui est conçue de telle sorte qu'à l'aide de l'unité de commutation, sélectivement au moins l'une des au moins trois électrodes soit commutable en tant qu'électrode de travail (WE, 210) couplée à une solution d'électrolyte d'un analyte électrolytique et qu'au moins deux des électrodes des au moins trois électrodes soient commutables sélectivement en tant que contre-électrode (CE) et dans lequel les au moins trois électrodes sont conçues de telle sorte que des événements de capteur se produisent sur une électrode commutée en tant qu'électrode de travail (WE, 210) parmi les au moins trois électrodes dans la solution d'électrolyte en présence de l'analyte électrolytique, **caractérisé par** un circuit de commande qui est conçu pour la commande, la sélection et/ou la lecture des au moins trois électrodes ou d'une partie des au moins trois électrodes, afin de réaliser la contre-électrode (CE) par interconnexion d'électrodes du réseau de capteurs non utilisées en tant qu'électrodes de travail.

2. Ensemble de capteurs selon la revendication 1, dans lequel l'unité de commutation comporte au moins un transistor MOS qui est mis en oeuvre à l'intérieur du réseau de capteurs.

3. Ensemble de capteurs selon la revendication 1, l'ensemble de capteurs comprenant au moins un circuit de fonctionnement et l'unité de commutation étant mise en oeuvre à l'extérieur du réseau de capteurs et l'au moins un circuit de fonctionnement (208) des au moins deux électrodes commutées en tant que contre-électrode (CE) commune du réseau de capteurs pouvant être commandé au moyen de l'unité de commutation de telle sorte que le potentiel d'électrode suive le potentiel au niveau de la borne de contre-électrode d'un potentiostat.

4. Ensemble de capteurs selon l'une des revendications 1 à 3, avec de 50 à 1 000 000, de préférence avec de 100 à 250 000 électrodes.

5. Ensemble de capteurs selon l'une des revendications 1 à 4, dans lequel la pluralité des au moins trois électrodes sont conçues pour la détection de substances oxydables resp. réductibles.

6. Ensemble de capteurs selon l'une des revendications 1 à 5, conçu pour la détection de molécules d'acide nucléique, de peptides et/ou de protéines.

7. Ensemble de capteurs selon l'une des revendications 1 à 6, dans lequel des molécules à effet capteur sont immobilisées sur la surface des électrodes.

8. Ensemble de capteurs selon l'une des revendications 1 à 7 qui est conçu de telle sorte qu'en présence d'un analyte électrolytique, un signal de capteur peut être produit au moyen de marqueurs électrochimiquement actifs.

9. Ensemble de capteurs selon la revendication 8, dans lequel les marqueurs électrochimiquement actifs comprennent des marqueurs complexe métallique.

10. Ensemble de capteurs selon la revendication 8 ou la revendication 9, dans lequel les marqueurs électrochimiquement actifs comprennent des marqueurs ferrocène.

11. Procédé de fonctionnement d'un ensemble de capteurs qui comprend un réseau de capteurs et un substrat, dans lequel le réseau de capteurs est intégré monolithiquement dans et/ou sur le substrat, dans un procédé d'analyse électrochimique, dans lequel l'ensemble de capteurs comprend au moins trois électrodes, la pluralité desquelles sont conçues sous la forme d'ensembles de biocapteurs pour la détection de biomolécules, et une unité de commutation qui est conçue de telle sorte qu'à l'aide de l'unité de commutation, sélectivement au moins l'une des au moins trois électrodes soit commutable en tant qu'électrode de travail (WE, 210) couplée à une solution d'électrolyte d'un analyte électrolytique et qu'au moins deux des électrodes soient commutables sélectivement en tant que contre-électrode (CE) et dans lequel les au moins trois électrodes sont conçues de telle sorte que des événements de capteur se produisent sur une électrode commutée en tant qu'électrode de travail (WE, 210) parmi les au moins trois électrodes dans la solution d'électrolyte en présence de l'analyte électrolytique, dans lequel selon le procédé
(a) un premier nombre des au moins trois électrodes, qui est constitué d'au moins deux électrodes, sont interconnectées, sont reliées à la borne de contre-électrode d'un potentiostat et la contre-électrode du réseau de capteurs est ainsi réalisée ;
(b) un deuxième nombre des au moins trois électrodes, qui est constitué d'au moins une électrode, sont commutées en tant qu'électrodes de travail (WE, 210) pour la détection d'un analyte électrolytique ;
(c) après détection des événements de capteur au niveau des électrodes de travail (WE, 210) dans (b), au moins une électrode du premier nombre des au moins trois électrodes est commutée en tant qu'électrode de travail (WE, 210) pour la détection d'un analyte électrolytique ;
(d) au moins une électrode du deuxième nombre des au moins trois électrodes selon (a) est commutée en tant que contre-électrode (CE) ; et
(e) les étapes (a) à (d) peuvent être répétées un nombre de fois quelconque.

12. Procédé de fonctionnement d'un ensemble de capteurs dans un procédé d'analyse électrochimique selon la revendication 11, dans lequel la surface totale des électrodes commutées en tant que contre-électrodes (CE) dans les étapes (a) et (d) est au moins 10 fois plus grande que la surface totale des électrodes commutées en tant qu'électrodes de travail (WE, 210).

13. Procédé de fonctionnement d'un ensemble de capteurs dans un procédé d'analyse électrochimique selon la revendication 11 ou la revendication 12, dans lequel au moins une substance dans un électrolyte est déterminée qualitativement et/ou quantitativement sur la base de sa tension d'oxydation resp. de réduction caractéristique au moyen de la voltamétrie, de l'ampérométrie et/ou de la coulométrie.

14. Procédé de fonctionnement d'un ensemble de capteurs dans un procédé d'analyse électrochimique selon l'une des revendications 11 à 13, dans lequel pour l'analyse de biomolécules, des molécules à effet capteur marquées par du ferrocène sont immobilisées sur les électrodes et la concentration de la biomolécule est déterminée par mesure de la quantité de charge circulant lors de l'oxydation du ferrocène.

15. Procédé de fonctionnement d'un ensemble de capteurs dans un procédé d'analyse électrochimique selon l'une des revendications 11 à 14, dans lequel une contre-électrode externe est en outre utilisée pour l'amélioration du comportement temporel de l'ensemble de capteurs.

16. Procédé de fonctionnement d'un ensemble de capteurs dans un procédé d'analyse électrochimique selon l'une des revendications 11 à 15, dans lequel l'ensemble de capteurs comprend au moins un circuit de fonctionnement pour la commande des électrodes et le circuit de fonctionnement (208) d'une électrode de travail (WE, 210) est désactivé pour commutation en tant que contre-électrode (CE) au moyen de l'unité de commutation.
